# FASCICULE DE BREVET EUROPEEN

(11) **EP 1 464 328 B1**
(45) Date de publication et mention de la délivrance du brevet: **04.06.2008**
(21) Numéro de dépôt: 04290871.5
(22) Date de dépôt: 01.04.2004
(51) Int. Cl.: A61Q 5/02, A61Q 5/06, A61Q 5/08, A61Q 5/10, A61Q 5/12, A61K 8/31, A61K 8/37, A61K 8/49, A61K 8/92

(54) **Composition de coloration pour matières kéranitiques humaines comprenant un colorant fluorescent soluble et un agent conditionneur insoluble, procédé et utilisation**
Färbemittel für menschliche Keratinfasern, enthaltend einen löslichen fluoreszierenden Farbstoff sowie ein unlösliches Konditionierungsmittel, Verfahren und Verwendung
Composition for dyeing human keratinic fibres comprising a soluble fluorescent colouring agent and an insoluble conditioning agent, process and use

(30) Priorité: 01.04.2003 FR 0304021
(43) Date de publication de la demande: 06.10.2004
(73) Titulaire: L'ORÉAL, 75008 Paris (FR)
(72) Inventeur: Plos, Grégory, 75011 Paris (FR); Samain, Henri, 91570 Bièvres (FR)
(74) Mandataire: Wattremez, Catherine

(56) Documents cités:
- EP-A- 0 370 470
- WO-A-99/13828
- AT-B- 302 534
- US-A- 3 658 985

## Description

L'invention concerne une composition comprenant au moins un colorant fluorescent dans la gamme des oranges soluble et au moins un agent conditionneur insoluble particulier, ainsi que les procédés et dispositif mettant en oeuvre ces compositions. Elle concerne également l'utilisation de ces compositions pour colorer avec un effet éclaircissant les matières kératiniques humaines et plus particulièrement les fibres kératiniques comme les cheveux pigmentés ou colorés artificiellement.

Dans le domaine capillaire, il existe principalement deux grands types de coloration capillaire.
Le premier est la coloration semi-permanente ou coloration directe qui fait appel à des colorants capables d'apporter à la coloration naturelle des cheveux, une modification plus ou moins marquée résistant à plusieurs shampooings. Ces colorants sont appelés colorants directs et peuvent être mis en oeuvre de deux manières différentes. Les colorations peuvent être réalisées par application directe sur les fibres kératiniques de la composition contenant le ou les colorants directs ou par application d'un mélange réalisé extemporanément d'une composition contenant le ou les colorants directs avec une composition contenant un agent décolorant oxydant qui est de préférence l'eau oxygénée. On parle alors de coloration directe éclaircissante.
Le deuxième est la coloration permanente ou coloration d'oxydation. Celle-ci est réalisée avec des précurseurs de colorants dits "d'oxydation" qui sont des composés incolores ou faiblement colorés qui une fois mélangés à des produits oxydants, au moment de l'emploi, peuvent donner naissance par un processus de condensation oxydative à des composés colorés et colorants. Il est souvent nécessaire d'associer aux bases d'oxydation et coupleurs, un ou plusieurs colorants directs afin de neutraliser ou de rabattre les nuances trop en reflets rouges, orangés ou dorés, ou au contraire d'accentuer ces reflets rouges, orangés ou dorés.
Parmi les colorants directs disponibles, les colorants directs nitrés benzéniques ne sont pas suffisamment puissants, les indoamines, les colorants quinoniques ainsi que les colorants naturels présentent une faible affinité pour les fibres kératiniques et de ce fait conduisent à des colorations qui ne sont pas assez résistantes vis à vis des différents traitements que peuvent subir les fibres, et en particulier vis à vis des shampooings.

En outre, il existe un besoin d'obtenir un effet d'éclaircissement des fibres kératiniques humaines. Cet éclaircissement est obtenu classiquement par un procédé de décoloration des mélanines du cheveu par un système oxydant, généralement constitué par du peroxyde d'hydrogène associé ou non à des persels. Ce système de décoloration présente l'inconvénient de dégrader les fibres kératiniques et d'altérer leurs propriétés cosmétiques.

La présente invention a pour objet de résoudre les problèmes mentionnés ci-dessus et notamment de proposer une composition qui présente une bonne affinité tinctoriale pour les matières kératiniques et notamment les fibres kératiniques, de bonnes propriétés de ténacité vis à vis des agents extérieurs, et en particulier vis-à-vis des shampooings, et qui permettent également d'obtenir un éclaircissement sans altération de la matière traitée, plus particulièrement la fibre kératinique.

Il a donc été trouvé de façon inattendue et surprenante que l'utilisation de colorants fluorescents, dans la gamme des orangés solubles, en présence d'agents conditionneurs insolubles particuliers, permettait d'atteindre ces objectifs.

La présente invention a donc pour premier objet une composition comprenant, dans un milieu cosmétiquement acceptable, au moins un colorant fluorescent dans la gamme des orangés soluble dans ledit milieu et au moins un agent conditionneur insoluble dans ledit milieu, choisi parmi les huiles de synthèse ; les huiles minérales ; les huiles végétales ; les huiles animales ; les huiles fluorées ou perfluorées ; les cires naturelles ou synthétiques ; les esters d'acides carboxyliques ; les composés de formule R₃CHOH-CH(NHCOR₁)-CH₂OR₂ dans laquelle R₁ est un radical alkyle en C₁₄-C₃₀, éventuellement substitué en position α par un radical hydroxyle, ou en position ω par un radical hydroxyle estérifié par un acide gras en C₁₆-C₃₀, R₂ désigne un atome d'hydrogène ou un radical (glycosyle)n ou (galactosyle)m avec n variant de 1 à 4 et m de 1 à 8, R désigne un radical hydrocarboné en C₁₅-C₂₆ éventuellement substitué par un radical alkyle, ou un radical α-hydroxytalkyle en C₁₅-C₂₆ éventuellement estérifié par un α-hydroxyacide en C₁₆-C₃₀ ; seuls ou en mélanges.

Un second objet de l'invention concerne un procédé pour colorer avec un effet éclaircissant les cheveux présentant une hauteur de ton inférieure ou égale à 6, dans lequel on met en oeuvre les étapes suivantes :
a) on applique sur lesdits cheveux une composition selon l'invention, pendant un temps suffisant pour développer la coloration et l'éclaircissement désirés,
b) on rince éventuellement les cheveux,
c) éventuellement on lave au shampooing et on rince les cheveux,
d) on sèche ou on laisse sécher les cheveux.

Un autre objet de l'invention concerne l'utilisation de la composition pour colorer avec un effet éclaircissant les cheveux présentant une hauteur de ton inférieure ou égale à 6.

Un dispositif à plusieurs compartiments pour la coloration et l'éclaircissement des fibres kératiniques, comprenant au moins un compartiment renfermant la composition selon l'invention, et au moins un autre compartiment renfermant une composition renfermant au moins un agent oxydant, constitue un autre objet de l'invention.

Les compositions de l'invention permettent en particulier une meilleure fixation du colorant fluorescent dans les matières kératiniques ce qui se traduit par un effet de fluorescence accru et à un effet d'éclaircissement supérieur à celui obtenu avec le colorant fluorescent utilisé seul.
On constate également une meilleure ténacité du résultat vis-à-vis des lavages ou shampooings.

Mais d'autres caractéristiques et avantages de la présente invention apparaîtront plus clairement à la lecture de la description et des exemples qui vont suivre.

A moins d'une indication différente, les bornes des gammes de valeurs qui sont données dans la description, sont incluses dans ces gammes.

Comme cela a été indiqué auparavant, la composition selon l'invention comprend au moins un colorant fluorescent soluble et au moins un agent conditionneur insoluble particulier.

Par colorant fluorescent, on entend au sens de la présente invention un colorant qui est une molécule qui colore par elle-même, et donc absorbe la lumière du spectre visible et éventuellement de l'ultraviolet (longueurs d'onde allant de 360 à 760 nanomètres) mais qui, contrairement à un colorant classique, transforme l'énergie absorbée en lumière fluorescente de plus grande longueur d'onde émise dans la partie visible du spectre.

Un colorant fluorescent selon l'invention est à différencier d'un agent éclaircissant optique. Les agents éclaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores, qui ne colorent pas, car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Enfin, le colorant fluorescent mis en oeuvre dans la composition est soluble dans le milieu de la composition. Précisons que le colorant fluorescent est différent en cela d'un pigment fluorescent qui lui, n'est pas soluble dans le milieu de la composition.

Plus particulièrement, le colorant fluorescent utilisé dans le cadre de la présente invention, éventuellement neutralisé, est soluble dans le milieu de la composition à au moins 0,001 g/l, plus particulièrement au moins 0,5 g/l, de préférence au moins 1 g/l et selon un mode de réalisation encore plus préféré, au moins 5 g/l à la température comprise entre 15 et 25°C.

A titre d'exemples de colorants fluorescents susceptibles d'être mis en oeuvre, on peut citer les colorants fluorescents appartenant aux familles suivantes : les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents monocationiques ou polycationiques de type azoïque, azométhinique, ou méthinique, seuls ou en mélanges ; de préférence aux familles suivantes : les naphtalimides ; les coumarines cationiques ou non ; les azaxanthènes ; les naphtolactames ; les azlactones ; les oxazines ; les thiazines ; les dioxazines ; les colorants fluorescents monocationiques ou polycationiques de type azoïque, azométhinique, ou méthinique, seuls ou en mélanges.

Les colorants fluorescents utilisés selon la présente invention sont des colorants dans la gamme des orangés.

De préférence, les colorants fluorescents de l'invention conduisent à un maximum de réflectance se situant dans la gamme de longueur d'onde allant de 500 à 650 nanomètres, et de préférence dans la gamme de longueur d'onde allant de 550 à 620 nanomètres.

Certains des colorants fluorescents selon la présente invention sont des composés connus en eux-mêmes.

On peut notamment citer parmi eux :
- le Jaune Brilliant B6GL commercialisé par la société SANDOZ et de structure suivante : 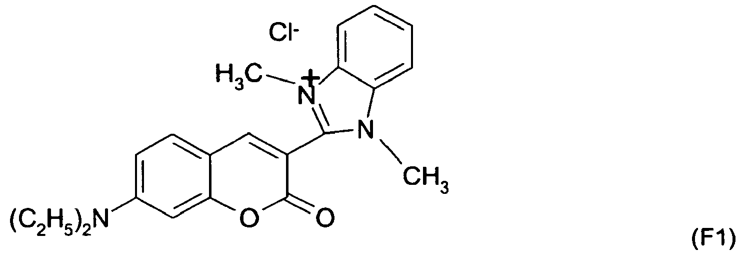
- le Basic Yellow 2, ou Auramine O commercialisé par les sociétés PROLABO, ALDRICH ou CARLO ERBA et de structure suivante : 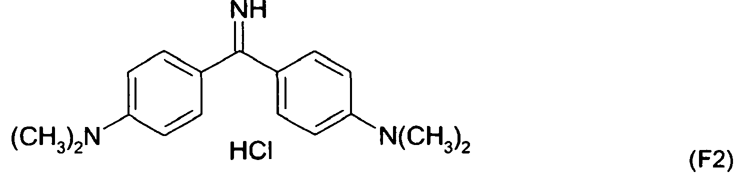 monochlorhydrate de 4,4'-(imidocarbonyl)bis(N,N-diméthylaniline) - CAS number 2465-27-2.

On peut aussi citer les composés de formule suivante : 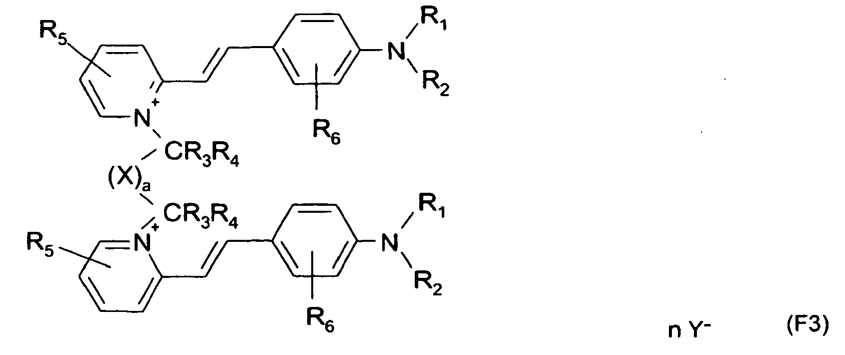 dans laquelle :
R₁, R₂, identiques ou différents, représentent :
   - un atome d'hydrogène ;
   - un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
   - un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
   - R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
   - R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
   - un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
   - un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
   - un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
   - un radical dicarbonyle ;
   - le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent.

Rappelons que les termes hétéroatomes, représentent un atome d'oxygène ou d'azote.
Parmi les groupements porteurs de tels atomes, on peut citer entre autres les groupements hydroxyle, alcoxy, carbonyle, amino, ammonium, amido (-N-CO-), carboxyle (-O-CO- ou -CO-O-).
En ce qui concerne les groupements alcényles, ces derniers comprennent une ou plusieurs liaisons carbone-carbone insaturée (-C=C-), et de préférence une seule double liaison carbone-carbone.

Dans cette formule générale, les radicaux R₁ et R₂, identiques ou non, représentent plus particulièrement :
- un atome d'hydrogène ;
- un radical alkyle comprenant 1 à 10 atomes de carbone, notamment 1 à 6 atomes de carbone, de préférence 1 à 4 atomes de carbone, éventuellement interrompu par un atome d'oxygène ou éventuellement substitué par au moins un radical hydroxyle, amino, ammonium, d'un atome de chlore ou de fluor ;
- un radical benzyle, phényle, éventuellement substitué par un radical alkyle ou alcoxy comprenant 1 à 4 atomes de carbone, de préférence 1 ou 2 atomes de carbone ;
- avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire
ou ramifié comprenant 1 à 4 atomes de carbone éventuellement interrompu et/ou substitué par un atome d'azote et/ou d'oxygène et/ou groupement portant un atome d'azote et/ou d'oxygène.

En ce qui concerne les radicaux amino ou ammonium précités, les radicaux portés par l'atome d'azote peuvent ou non être identiques et représenter plus particulièrement un atome d'hydrogène, un radical alkyle en C₁-C₁₀, de préférence en C₁-C₄, un radical arylalkyle dans lequel, plus spécialement, le radical aryle comprend 6 atomes de carbone et le radical alkyle 1 à 10 atomes de carbone, de préférence 1 à 4 atomes de carbone.

Selon un mode de réalisation avantageux de l'invention, les radicaux R₁ et R₂, identiques ou non, représentent un atome d'hydrogène ; un radical alkyle linéaire ou ramifié en C₁-C₆ ; un radical alkyle en C₂-C₆ substitué par un radical hydroxyle ; un radical alkyle en C₂-C₆ portant un groupement amino ou ammonium ; un radical chloroalkyle en C₂-C₆ ; un radical alkyle C₂-C₆ interrompu par un atome d'oxygène ou groupement en portant un (par exemple ester) ; un radical aromatique comme les phényle, benzyle, 4-méthylphényle ; un radical hétérocyclique tel que les radicaux pyrrolo, pyrrolidino, imidazolo, imidazolino, imidazolium, pipérazino, morpholo, morphollino, pyrazolo, triazolo, éventuellement substitué par au moins un radical alkyle en C₁-C₆ ou aromatique.

De préférence, les radicaux R₁ et R₂, identiques ou différents, représentent un atome d'hydrogène, un radical alkyle linéaire ou ramifié en C₁-C₆ tels que les radicaux méthyle, éthyle, n-butyle, n-propyle ; le 2-hydroxyéthyle ; un radical alkyltriméthylammonium ou alkyltriéthylammonium, le radical alkyle étant linéaire en C₂-C₆ ; un radical (di)alkylméthylamino ou (di)alkyléthylamino, le radical alkyle étant linéaire en C₂-C₆ ; -CH₂CH₂Cl ; -(CH₂)ₙ-OCH₃ ou -(CH₂)ₙ-OCH₂CH₃ avec n nombre entier variant de 2 à 6 ; -CH₂CH₂-OCOCH₃ ; -CH₂CH₂COOCH₃.
De préférence les radicaux R₁ et R₂, identiques ou non, et de préférence identiques, représentent un radical méthyle, un radical éthyle.

Les radicaux R₁ et R₂, identiques ou différents, peuvent aussi représenter un radical hétérocyclique du type pyrrolidino, 3-amino pyrrolidino, 3-(diméthyl)amino pyrrolidino, 3-(triméthyl)amino pyrrolidino, 2,5-diméthylpyrrolo, le 1H-imidazole, 4-méthyl pipérazino, 4-benzyl pipérazino, morpholo, 3,5-(ter-butyl)-1H-pyrazolo, 1H-pyrazolo, 1H-1,2,4-triazolo.

Les radicaux R₁ et R₂, identiques ou différents, peuvent aussi représenter être reliés de manière à former un hétérocycle de formules (I) et (II) suivantes : 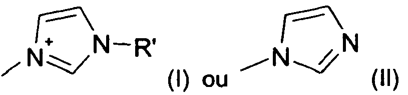 dans lesquelles R' représente un atome d'hydrogène, un radical alkyle en C₁-C₃, -CH₂CH₂OH, -CH₂CH₂OCH₃.

Conformément à un mode de réalisation plus particulier de l'invention, R₅, identiques ou non, représentent un atome d'hydrogène, un atome de fluor ou de chlore, un radical alkyle linéaire ou ramifié comprenant 1 à 4 atomes de carbone éventuellement interrompu par un atome d'oxygène ou d'azote.
Il est précisé que le substituant R₅, s'il est différent de l'hydrogène, se trouve avantageusement en position(s) 3 et/ou 5 par rapport au carbone du cycle portant l'azote substitué par les radicaux R₁ et R₂, et de préférence en position 3 par rapport à ce carbone.
Avantageusement, les radicaux R₅, identiques ou non, représentent un atome d'hydrogène ; un radical alkyle linéaire ou ramifié en C₁-C₄ ; -O-R₅₁ avec R₅₁ représentant un radical alkyle linéaire en C₁-C₄ ; -R₅₂-O-CH₃ avec R₅₂ représentant un radical alkyle linéaire en C₂-C₃ ; -R₅₃ - N (R₅₄)₂ dans laquelle R₅₃ représente un radical alkyle linéaire en C₂-C₃, R₅₄, identiques ou différents, représentent un atome d'hydrogène ou un radical méthyle.
De préférence R₅, identiques ou non, représentent l'hydrogène, un méthyle, un méthoxy, et de préférence, R₅ représente un atome d'hydrogène.

Selon un mode de réalisation particulier, les radicaux R₆, identiques ou différents, représentent un atome d'hydrogène ; un radical alkyle linéaire ou ramifié en C₁-C₄ ; -X avec X représentant un atome de chlore, de brome ou de fluor ; -R₆₁-O-R₆₂ avec R₆₁ représentant un radical alkyle linéaire en C₂-C₃ et R₆₂ représente le radical méthyle ; -R₆₃-N(R₆₄)₂ avec R₆₃ représentant un radical alkyle linéaire en C₂-C₃, R₆₄, identiques ou différents, représentent un atome d'hydrogène ou un radical méthyle, -N(R₆₅)₂ dans laquelle R₆₅, identiques ou différents, représentent un atome d'hydrogène, un radical alkyle linéaire en C₂-C₃ ; -NHCO R₆₆ avec R₆₆ représentant un radical alkyle en C₁-C₂, un radical chloroalkyle en C₁-C₂, un radical -R₆₇-NH₂ ou -R₆₇-NH(CH₃) ou -R6₇-N(CH₃)₂ ou -R₆₇-N⁺(CH₃)₃ ou -R₆₇-N⁺(CH₂CH₃)₃ avec R₆₇ représentant un radical alkyle en C₁-C₂.
Il est précisé que le substituant R₆, s'il est différent de l'hydrogène, se trouve de préférence en position 2 et/ou 4 par rapport à l'atome d'azote du cycle pyridinium, et de préférence en position 4 par rapport à cet atome d'azote.

Plus particulièrement ces radicaux R₆, identiques ou non, représentent un atome d'hydrogène ou un radical méthyle ou éthyle, et de préférence, R₆ représente un atome d'hydrogène.

En ce qui concerne les radicaux R₃, R₄, ces derniers, identiques ou non, représentent avantageusement un atome d'hydrogène, un radical alkyle comprenant 1 à 4 atomes de carbone, plus spécialement un radical méthyle. De manière préférée, R₃ et R₄ représentent chacun un atome d'hydrogène.

Comme indiqué plus haut, X représente :
- un radical alkyle, linéaire ou ramifié comprenant 1 à 14 atomes de carbone, ou alcényle comprenant 2 à 14 atomes de carbone, éventuellement interrompu et/ou substitué par au moins un hétéroatome, par au moins un groupement porteur d'au moins un hétéroatome et/ou par au moins un atome d'halogène ;
- un radical hétérocyclique comprenant 5 ou 6 chaînons, éventuellement substitué par au moins un radical alkyle linéaire ou ramifié comprenant 1 à 14 atomes de carbone, par au moins un radical aminoalkyle, linéaire ou ramifié, comprenant 1 à 4 atomes de carbone, éventuellement substitué par au moins un hétéroatome ; par au moins un atome d'halogène ;
- un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement comprenant au moins un hétéroatome ;
- un radical dicarbonyle.

En outre, il est indiqué que le groupement X peut porter une ou plusieurs charges cationiques.

Ainsi, X peut représenter un radical alkyle, linéaire ou ramifié, comprenant 1 à 14 atomes de carbone, ou alcényle comprenant 2 à 14 atomes de carbone, et peut être substitué et/ou interrompu par un ou plusieurs atomes d'oxygène et/ou d'azote, et/ou par un ou plusieurs groupements porteurs d'au moins un hétéroatome , et/ou par un atome de fluor, de chlore.
Parmi les groupements de ce type, on peut citer tout particulièrement les groupements hydroxyle, alcoxy (avec notamment un radical R de type alkyle en C₁-C₄), amino, ammonium, amido, carbonyle, carboxyle (-COO-, -O-CO-) avec notamment un radical de type alkyloxy.
Notons que l'atome d'azote, s'il est présent, peut se trouver sous une forme quaternisée ou non. Dans ce cas, le ou les deux autres radicaux portés par l'atome d'azote quaternisé ou non, sont identiques ou non et peuvent être un atome d'hydrogène, un radical alkyle en C₁-C₄, de préférence le méthyle.

Selon une autre variante, le groupement X représente un radical hétérocyclique comprenant 5 ou 6 chaînons, du type imidazolo, pyrazolo, triazino, pyridino, éventuellement substitué par au moins un radical alkyle linéaire ou ramifié comprenant 1 à 14 atomes de carbone, plus particulièrement 1 à 10 atomes de carbone, de préférence de 1 à 4 atomes de carbone ; par au moins un radical aminoalkyle, linéaire ou ramifié, comprenant 1 à 10 atomes de carbone, de préférence de 1 à 4 atomes de carbone, éventuellement substitué par un groupement comprenant au moins un hétéroatome (de préférence un radical hydroxyle), ou par un atome d'halogène. A noter que le groupement amino est de préférence lié à l'hétérocycle.

Conformément à une autre possibilité, le groupement X représente un radical aromatique (comprenant de préférence 6 atomes de carbone) ou diaromatique condensé ou non (comprenant notamment de 10 à 12 atomes de carbone), séparé ou non par un radical alkyle comprenant 1 à 4 atomes de carbone, le ou les radicaux aryles étant éventuellement substitués par au moins au moins un atome d'halogène et/ou par au moins un radical alkyle comprenant 1 à 10 atomes de carbone, de préférence 1 à 4 atomes de carbone, éventuellement interrompu par au moins un atome d'oxygène et/ou d'azote, et/ou groupement comprenant au moins un hétéroatome (comme un radical carbonyle, carboxyle, amido, amino, ammonium).
Il est à noter que le radical aromatique, de préférence un radical phényle, est relié aux groupements CR₃R₄ par l'intermédiaire de liaisons en positions 1,2 ; 1,3 ou 1;4, de préférence en positions 1,3 et 1,4. Si le radical phényle relié par l'intermédiaire de liaisons en positions 1,4, porte un ou deux substituants, ce ou ces derniers sont situés de préférence en position 1,4 par rapport à l'un des groupements CR₃R₄. Si le radical phényle relié par l'intermédiaire de liaisons en positions 1,3, porte un ou deux substituants, ce ou ces derniers sont situés de préférence en position 1 et/ou 3 par rapport à l'un des groupements CR₃R₄.

Au cas où le radical est diaromatique, il est de préférence non condensé et comprend deux radicaux phényles séparés ou non par une liaison simple (soit un carbone de chacun des deux cycles) ou par un radical alkyle, de préférence de type CH₂ ou C(CH₃)₂. De manière préférée, les radicaux aromatiques ne portent pas de substituant.
Il est à noter que ledit radical diaromatique est relié aux groupements CR₃R₄ par l'intermédiaire de liaisons en positions 4,4'.

A titre d'exemples de groupements X convenables, on peut citer notamment les radicaux alkyle linéaires ou ramifiés comprenant 1 à 13 atomes de carbone tels que méthylène, éthylène, propylène, isopropylène, n-butylène, pentylène, hexylène ; le 2-hydroxypropylène, le 2-hydroxy n-butylène ; les radicaux alkylènes en C₁-C₁₃, substitués ou interrompus par un ou plusieurs atomes d'azote et/ou d'oxygène, et/ou groupements portant au moins un hétéroatome (hyroxyle, amino, ammonium, carbonyle, carboxyle, par exemple) tels que -CH₂CH₂OCH₂CH₂-, le 1,6-didéoxy-d-mannitol, -CH₂N⁺(CH₃)₂CH₂-, -CH₂CH₂N⁺(CH₃)₂-(CH₂)₆N⁺(CH₃)₂-CH₂CH₂-, CO-CO-, le 3,3-diméthylpentylène, le 2-acétoxyéthylène, le butylène1,2,3,4 tétraol ; -CH=CH- ; les radicaux aromatiques ou diaromatiques substitués par un ou plusieurs radicaux alkyle, par un ou plusieurs groupements portant au moins un hétéroatome et/ou par un ou plusieurs atomes d'halogène, tels que le 1,4-phénylène, le 1,3-phénylène, le 1,2-phénylène, le 2,6-fluorobenzène, le 4,4'-biphénylène, le 1,3-(5-méthyl benzène), le 1,2-bis(2-méthoxy)benzène, le bis(4-phényl)méthane, le 3,4 benzoate de méthyle, le 1,4-bis(amido méthyl)phényle; les radicaux de type hétérocycliques comme la pyridine, ou dérivé tel que le 2,6-bispyridine, l'imidazole, l'imidazolium, la triazine.

X représente, selon un mode de réalisation plus particulier de l'invention, un radical alkyle linéaire ou ramifié en C₁-C₁₃ ; -CH₂CH(OH)CH₂- ; -CH₂CH(Cl)CH₂- ; -CH₂CH₂-OCOCH₂- ; -CH₂CH₂COOCH₂- ; -Ra-O- Rb- avec Ra représentant un radical alkyle linéaire en C₂-C₆ et Rb représente un radical alkyle linéaire en C₁-C₂ ; - Rc-N(Rd)-Re-avec Rc représentant un radical alkyle en C₂-C₉, Rd représentant un atome d'hydrogène, un radical alkyle en C₁-C₂ et Re représentant un radical alkyle en C₁-C₆ ; -Rf-N⁺(Rg)₂-Rh- avec Rf représentant un radical alkyle linéaire en C₂-C₉, Rg, de préférence identiques, représentent un radical alkyle en C₁-C₂, Rh représente un radical alkyle linéaire en C₁-C₆ ; -CO-CO-.

X peut de plus représenter un radical imidazole, éventuellement substitué par au moins un radical alkyle comprenant 1 à 14 atomes de carbone, plus particulièrement 1 à 10 atomes de carbone, de préférence de 1 à 4, et par exemple les radicaux divalents de formule suivante : 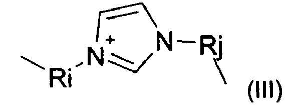
dans laquelle Ri et Rj, identiques ou non, représentent un radical alkyle linéaire en C₁-C₆
X peut de même être choisi parmi les radicaux divalents dérivés de triazine suivants :

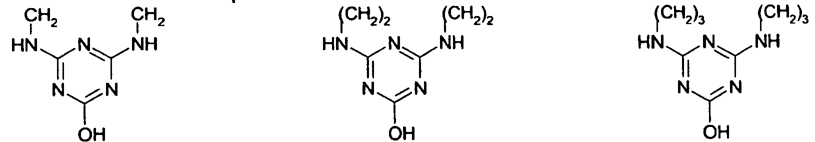

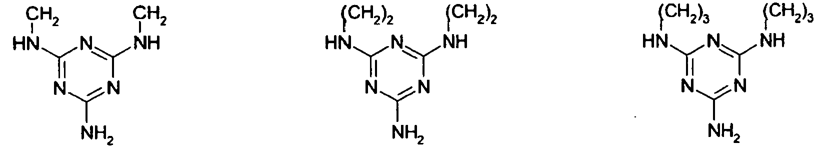

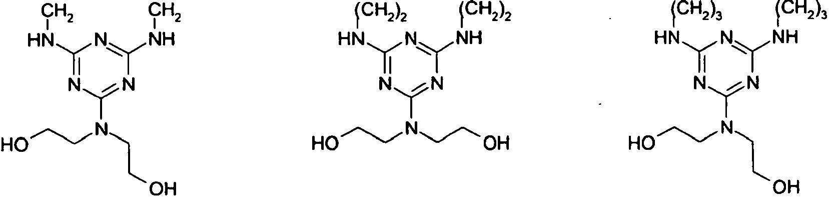

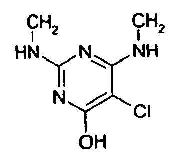

Selon une autre possibilité, X peut représenter les radicaux divalents aromatiques suivants :

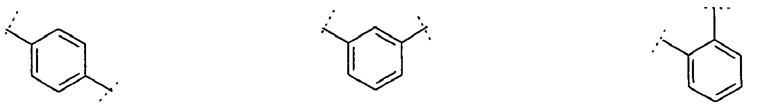

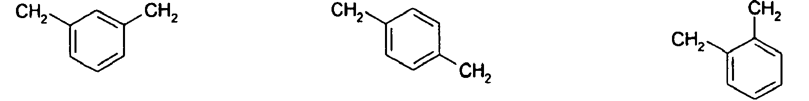

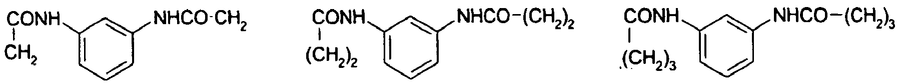

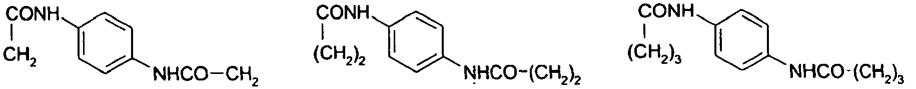

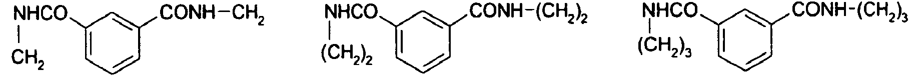

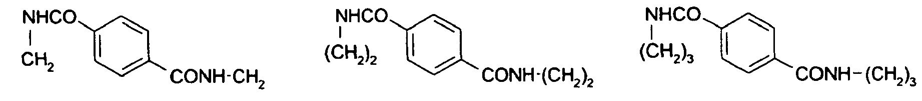

Dans la formule générale de ces composés fluorescents, Y- représente un anion organique ou minéral. S'il y a plusieurs anions Y-, ces derniers peuvent ou non être identiques.

Parmi les anions d'origine minérale, on peut citer sans intention de s'y limiter les anions provenant d'atomes d'halogène, tels que les chlorures de préférence, les iodures, les sulfates ou bisulfates, les nitrates, les phosphates, les hydrogénophosphates, les dihydrogénophosphates, les carbonate, les bicarbonates.
Parmi les anions d'origine organique, on peut citer les anions provenant des sels d'acides mono- ou polycarboxyliques, sulfoniques, sulfuriques, saturés ou non, aromatiques ou non, éventuellement substitués par au moins un radical hydroxyle, amino, ou atomes d'halogène. A titre d'exemples non limitatifs, conviennent les acétates, hydroxyactétates, aminoacétates, (tri)chloroacétates, benzoxyactétates, propionates et dérivés portant un atome de chlore, fumarates, oxalates, acrylates, malonates, succinates, lactates, tartrates, glycollates citrates, les benzoates et dérivés portant un radical méthyle ou amino, les alkylsulfates, les tosylates, les benzènesulfonates, toluènesulfonates.

De préférence, le ou les anions Y, identiques ou non, sont choisis parmi le chlore, le sulfate, le méthosulfate, l'éthosulfate.

Enfin, le nombrren, entier, est au moins égal à 2 et au plus égal au nombre de charges cationiques présentes dans le composé fluorescent.

De préférence les composés fluorescents qui viennent d'être détaillés sont des composés symétriques.

Ces composés peuvent être synthétisés en mettant en faisant réagir dans une première étape de α-picoline avec un réactif comprenant deux groupes partant qui peuvent être choisis parmi les atomes d'halogène, de préférence le brome, éventuellement le chlore, ou les groupements de type tolylsulfonyle ou méthylesulfonyle.
Cette première étape peut avoir lieu en présence d'un solvant, bien qu'il ne soit pas obligatoire, comme par exemple le diméthylformamide.
Le nombre de moles d'α-picoline est en général voisin de 2 pour une mole de réactif comprenant les groupes partant.
En outre, la réaction est habituellement mise en oeuvre au reflux du réactif et/ou du solvant s'il est présent.
Le produit issu de cette première étape est ensuite contacté avec un aldéhyde correspondant de formule suivante : 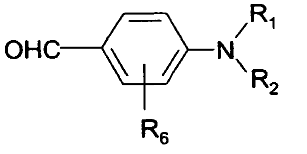 dans laquelle R₁, R₂ et R₆ ont les mêmes significations que précédemment indiquées.
Là encore, la réaction peut être effectuée en présence d'un solvant approprié, de préférence au reflux.
Il est à noter que les radicaux R₁ et R₂ de l'aldéhyde peuvent avoir la signification indiquée dans la formule générale détaillée auparavant.
Il est aussi possible de mettre en oeuvre un aldéhyde pour lequel lesdits radicaux représentent des atomes d'hydrogène et effectuer conformément à des méthodes classiques, la substitution de ces atomes d'hydrogène par des radicaux appropriés tels que décrits dans la formule générale une fois la deuxième étape terminée.

On pourra notamment se référer à des synthèses telles que décrites dans US 4256458.

On peut de même utiliser les composés de structure suivante : 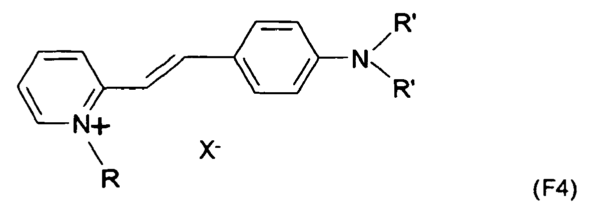 formule dans laquelle R représente un radical méthyle ou éthyle; R' représente un radical méthyle, X- un anion du type chlorure, iodure, sulfate, méthosulfate, acétate, perchlorate ;
A titre d'exemple de composé de ce type on peut citer le Photosensitiving Dye NK-557 commercialisé par la société UBICHEM, pour lequel R représente un radical éthyle, R' un radical méthyle et X- un iodure.

De préférence, les colorants fluorescents sont choisis parmi les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents monocationiques ou polycationiques de type azoïque, azométhinique, ou méthinique, seuls ou en mélanges ; de préférence les naphtalimides ; les coumarines cationiques ou non ; les azaxanthènes ; les naphtolactames ; les azlactones ; les oxazines ; les thiazines ; les dioxazines ; les colorants fluorescents monocationiques ou polycationiques de type azoïque, azométhinique, ou méthinique, seuls ou en mélanges.
A titre d'exemples de tels composés, on peut citer les composés de formules (F1) à (F3). Selon une variante préférée, la composition ne comprend pas de composé de formule (F4).

Le ou les colorants fluorescents présents dans la composition selon l'invention représentent avantageusement de 0,01 à 20 % en poids, plus particulièrement de 0,05 à 10 % en poids, et de préférence de 0,1 à 5% en poids, du poids total de la composition.

Comme indiqué auparavant, la composition selon l'invention comprend, outre le colorant fluorescent, au moins un agent conditionneur insoluble dans le milieu de la composition.

Il est tout d'abord rappelé que dans le cadre de la présente demande, on entend par agent conditionneur tout agent ayant pour fonction l'amélioration des propriétés cosmétiques des matières kératiniques telles que les cheveux, en particulier la douceur, le démêlage, le toucher, le lissage, l'électricité statique.

En outre, par insoluble dans le milieu de la composition, on entend tout composé qui dans tout ou partie d'un domaine de concentration compris entre 0,01 et 20 % en poids, à température ambiante, dans le milieu de la composition, ne forme pas, dans ces conditions, de solution macroscopiquement isotrope transparente.

Il est à noter que conformément à un mode de réalisation particulier de l'invention, les agents conditionneurs insolubles se trouvent notamment sous une forme dispersée dans le milieu de la composition sous forme de particules ayant généralement une taille moyenne en nombre comprise entre 2 nanomètres et 100 microns, de préférence entre 30 nanomètres et 20 microns (mesurée avec un granulomètre).

Ainsi, l'agent conditionneur est plus particulièrement choisi parmi les huiles de synthèse ; les huiles minérales ; les huiles végétales ; les huiles animales ; les huiles fluorées ou perfluorées ; les cires naturelles ou synthétiques ; les esters d'acides carboxyliques ; les composés de formule R₃CHOH-CH(NHCOR₁)-CH₂OR₂ ; seuls ou en mélanges.
De préférence l'agent conditionneur est choisi parmi les huiles de synthèse ; les huiles végétales ; les huiles animales ; les huiles fluorées ou perfluorées ; les cires naturelles ou synthétiques ; les esters d'acides carboxyliques ; les composés de formule R₃CHOH-CH(NHCOR₁)-CH₂OR₂ ; seuls ou en mélanges.

Plus particulièrement, les huiles de synthèse sont les polyoléfines en particulier les poly-α - oléfines et plus particulièrement :
- de type polybutène, hydrogéné ou non, et de préférence polyisobutène, hydrogéné ou non.
   On utilise de préférence les oligomères d'isobutylène de masse moléculaire en poids inférieure à 1000 g/mol et leurs mélanges avec des polyisobutylènes de masse moléculaire en poids supérieure à 1000 g/mol et de préférence allant de 1000 et 15000 g/mol.
   A titre d'exemples de poly-α-oléfines utilisables, on peut plus particulièrement mentionner les polyisobutènes vendus sous le nom de Permethyl® 99 A, 101 A , 102 A , 104 A (n=16) et 106 A (n=38) par la Société Presperse Inc, ou bien encore les produits vendus sous le nom de Arlamol® HD (n=3) par la Société ICI (n désignant le degré de polymérisation),
- de type polydécène, hydrogéné ou non.
   De tels produits sont vendus par exemple sous les dénominations Ethylflo® par la société Ethyl Corp., et d'Arlamol® Pao par la société ICI.

Les huiles animales ou végétales sont choisies préférentiellement dans le groupe formé par les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, par exemple, l'huile de Purcellin ou la cire liquide de jojoba.
On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote.

Les cires sont des substances naturelles (animales ou végétales) ou synthétiques solides à température ambiante (20°-25°C). Elles sont insolubles dans l'eau, solubles dans les huiles.
Sur la définition des cires, on peut citer par exemple P.D. Dorgan, Drug and Cosmetic Industry, Decembre 1983, pp. 30-33.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la Société Bertin (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société Sophim sous la référence M82, les cires de polyéthylène ou de polyoléfines en général.

Selon la présente invention, les composés de formule R₃CHOH-CH(NHCOR₁)-CH₂OR₂ dans laquelle R₁ est un radical alkyle en C₁₄-C₃₀, éventuellement substitué en position α par un radical hydroxyle, ou en position ω par un radical hydroxyle estérifié par un acide gras en C₁₆-C₃₀, R₂ désigne un atome d'hydrogène ou un radical (glycosyle)n ou (galactosyle)m avec n variant de 1 à 4 et m de 1 à 8, R désigne un radical hydrocarboné en C₁₅-C₂₆ éventuellement substitué par un radical alkyle, ou un radical α-hydroxytalkyle en C₁₅-C₂₆ éventuellement estérifié par un α-hydroxyacide en C₁₆-C₃₀.
A titre d'exemple, on peut notamment citer les N-linoléyldihydrosphingosine, N-oléyldihydrosphingosine, N-palmitoyldihydrosphingosine, N-stéaroyldihydrosphingosine, la N-béhénoyldihydrosphingosine, ou leurs mélanges.

Les huiles fluorées sont par exemple les perfluoropolyéthers décrits notamment dans EP 486135 et les composés fluorohydrocarbonées décrites notamment dans WO 93/11103.

Le terme de composés fluorohydrocarbonés désigne des composés dont la structure chimique comporte un squelette carboné dont certains atomes d'hydrogène ont été substitués par des atomes de fluor.

Les huiles fluorées peuvent également être des fluorocarbures tels que des fluoramines par exemple la perfluorotributylamine, des hydrocarbures fluorés, par exemple le perfluorodécahydronaphtalène, des fluoroesters et des fluoroethers.
Les perfluoropolyéthers sont par exemple vendus sous les dénominations commerciales Fomblin par la société Montefluos et Krytox par la société Du Pont.

Parmi les composés fluorohydrocarbonés, on peut également citer les esters d'acides gras fluorés tels que le produits vendu sous la dénomination Nofable FO par la société Nippon Oil.

Les alcools gras peuvent être choisis parmi les alcools gras en C₈-C₂₂, linéaires ou ramifiés ; ils sont éventuellement oxyalkylénés avec 1 à 15 moles d'oxyde d'alkylène ou polyglycérolés avec 1 à 6 moles de glycérol. L'oxyde d'alkylène est de préférence l'oxyde d'éthylène et/ou de propylène.

Les esters d'acides carboxyliques sont en particulier les esters mono, di, tri ou tétracarboxyliq ues.

Les esters d'acides monocarboxyliques sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle.

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La teneur en agent conditionneur insoluble est avantageusement comprise entre 0,01 et 20 % en poids, par rapport au poids de la composition, et de préférence entre 0,1 et 10 % en poids, par rapport au poids de la composition.

Le milieu cosmétiquement acceptable est généralement constitué par de l'eau ou par un mélange d'eau et d'un ou plusieurs solvants organiques usuels.

Parmi les solvants convenables, on peut citer plus particulièrement, les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.

Les solvants usuels décrits ci-dessus, s'ils sont présents, représentent habituellement de 1 à 40 % en poids, plus préférentiellement de 5 à 30 % en poids, par rapport au poids total de la composition.

Le pH de la composition conforme à l'invention est généralement compris entre 3 et 12 environ, et de préférence entre 5 et 11 environ.
Il peut être ajusté à la valeur désirée au moyen d'agents acidifiants ou alcalinisants.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 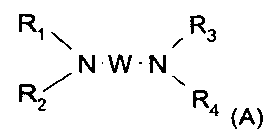 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ ou hydroxyalkyle en C₁-C₆.

Selon un mode de réalisation particulier de l'invention, la composition peut, comprendre, en plus du ou des colorants fluorescents, un ou plusieurs colorants directs additionnels non fluorescents, de nature non ionique, cationique ou anionique, qui peuvent par exemple être choisis parmi les colorants benzéniques nitrés.
Conviennent notamment les colorants directs benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-((β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le 1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

La composition conforme à l'invention peut également comprendre, en addition ou en remplacement de ces colorants benzéniques nitrés, un ou plusieurs colorants directs additionnels choisis parmi les colorants benzéniques nitrés jaunes, jaune-vert, bleus ou violets, les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ainsi que les colorants dérivés du triarylméthane, ou leurs mélanges.

Ces colorants directs additionnels peuvent notamment être des colorants basiques parmi lesquels on peut citer plus particulièrement les colorants connus dans le COLOR INDEX, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le COLOR INDEX, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans WO 95/01772, WO 95/15144 et EP 714954 et dont le contenu fait partie intégrante de la présente invention.

Parmi les colorants directs additionnels benzéniques nitrés jaunes et jaune-vert, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxybenzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-((3-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 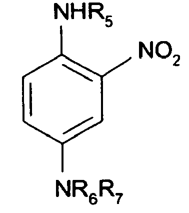
dans laquelle :
- R₆ représente un radical alkyle en C₁-C₄, un radical β-hydroxyéthyle ou β-hydroxypropyle ou γ-hydroxypropyle ;
- R₅ et R₇, identiques ou différents, représentent un radical β-hydroxyéthyle, -β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans FR 2692572.

Lorsqu'ils sont présents, le ou les colorants directs additionnels représentent de préférence de 0,0005 à 12 % en poids environ du poids total de la composition, et encore plus préférentiellement de 0,005 à 6 % en poids environ de ce poids.

Lorsqu'elle est destinée à la coloration d'oxydation, la composition conforme à l'invention comprend, en plus du ou des colorants fluorescents, au moins une base d'oxydation choisie parmi les bases d'oxydation classiquement utilisées pour la coloration d'oxydation et parmi lesquelles on peut notamment citer les paraphénylènediamines, les bis-phénylalkylènediamines, les para-aminophénols, les ortho-aminophénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylène diamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylène diamine, la N-(β-méthoxyéthyl) paraphénylènediamine et la 4'aminophényl 1-(3-hydroxy)pyrrolidine, et leurs sels d'addition avec un acide ou avec un agent alcalin.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide ou avec un agent alcalin.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut plus particulièrement citer à titre d'exemple, les dérivés pyridiniques, les dérivés pyrimidiniques et les dérivés pyrazoliques et leurs sels d'addition avec un acide ou avec un agent alcalin.

Lorsqu'elles sont utilisées, la ou les bases d'oxydation représentent avantageusement de 0,0005 à 12 % en poids par rapport au poids total de la composition, et de préférence de 0,005 à 6 % en poids environ de ce poids.

Lorsqu'elle est destinée à la coloration d'oxydation, la composition conforme à l'invention peut également comprendre, en plus du ou des colorants fluorescents et des bases d'oxydation, au moins un coupleur de façon à modifier ou à enrichir en reflets les nuances obtenues en mettant en oeuvre le(s) colorant(s) fluorescent(s) et la ou les bases d'oxydation.

Les coupleurs utilisables dans la composition conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent plus particulièrement de 0,0001 à 10 % en poids, et de préférence de 0,005 à 5 % en poids, par rapport au poids total de la composition.

D'une manière générale, les sels d'addition avec un acide utilisables dans le cadre des compositions de l'invention (bases d'oxydation et coupleurs) sont notamment choisis parmi les chlorhydrates, les bromhydrates, les sulfates, les citrates, les succinates, les tartrates, les tosylates, les benzènesulfonates, les lactates et les acétates.
Les sels d'addition avec un agent alcalin utilisables dans le cadre des compositions de l'invention (bases d'oxydation et coupleurs) sont notamment choisis parmi les sels d'addition avec les métaux alcalins ou alcalino-terreux, avec l'ammoniaque, avec les amines organiques dont les alcanolamines et les composés de formule (A).

La composition conforme à l'invention peut également comprendre divers adjuvants utilisés classiquement, tels que des agents tensioactifs anioniques, cationiques, non ioniques, amphotères, zwittérioniques ou leurs mélanges, des polymères anioniques, cationiques, non ioniques, amphotères, zwittérioniques autres que ceux de l'invention ou leurs mélanges, des agents épaississants minéraux, des agents antioxydants, des agents de pénétration, des agents séquestrants, des parfums, des tampons, des agents dispersants, des agents de conditionnement tels que par exemple des cations, des agents filmogènes, des agents conservateurs, des agents stabilisants, des agents opacifiants.

Parmi les agents épaississants, on préfère plus particulièrement utiliser les systèmes épaississants à base de polymères associatifs bien connus de l'homme de l'art et notamment de nature non ionique, anionique, cationique ou amphotère.

La teneur en agent tensioactif, de préférence de type non ionique, anionique ou encore amphotère, représente en général de 0,01 à 30 % en poids par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de liquides, de shampooings, de crèmes, de gels, ou sous toute autre forme appropriée.

Une forme particulièrement préférée selon la présente invention, la composition se trouve sous la forme d'un shampooing colorant et éclaircissant comprenant dans un milieu aqueux cosmétiquement acceptable.

Habituellement, le shampooing comprend au moins un agent tensioactif de type anionique, cationique, amphotère, ou non ionique. Parmi les agents tensioactifs non ioniques préférés on peut citer les alkylpolyglucosides.
Dans ces shampooings, l'agent tensioactif est présent dans une proportion allant d'environ 4 à 30 % et de préférence d'environ 8 à 20% en poids par rapport au poids total de la composition de shampooing.

Dans la composition selon l'invention, lorsqu'une ou plusieurs bases d'oxydation sont utilisées, éventuellement en présence d'un ou plusieurs coupleurs, ou lorsque le ou les colorants fluorescents sont utilisés dans le cadre d'une coloration directe éclaircissante, alors la composition conforme à l'invention peut en outre renfermer au moins un agent oxydant.
L'agent oxydant peut être choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux
ou à quatre électrons. L'utilisation du peroxyde d'hydrogène ou des enzymes est particulièrement préférée.

L'invention a également pour objet l'utilisation d'une composition telle que décrite ci-dessus pour colorer avec un effet éclaircissant des cheveux présentant une hauteur de ton inférieure ou égale à 6.

Selon la présente invention, on entend par matières kératiniques humaines, la peau, les cheveux, les ongles, les cils, et les sourcils, et plus particulièrement les cheveux pigmentés ou colorés artificiellement.

Au sens de l'invention, on entend par cheveux pigmentés ou colorés artificiellement, des cheveux dont la hauteur de ton est inférieure ou égale à 6 (blond foncé) et de préférence inférieure ou égale à 4 (châtain).
L'éclaircissement des cheveux est évalué par la 'hauteur de ton" qui caractérise le degré
ou le niveau d'éclaircissement. La notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage "Sciences des traitements capillaires" de Charles ZVIAK 1988, Ed.Masson, pp.215 et 278.
Les hauteurs de ton s'échelonnent de 1 (noir) à 10 (blond clair), une unité correspondant à un ton ; plus le chiffre est élevé et plus la nuance est claire.

Un autre objet de la présente invention concerne donc un procédé de coloration avec effet éclaircissant des cheveux présentant une hauteur de ton inférieure ou égale à 6, consistant à mettre en oeuvre les étapes suivantes :
a) on applique sur les cheveux, pendant un temps suffisant pour développer la coloration et l'éclaircissement désirés, la composition selon l'invention,
b) on rince éventuellement lesdits cheveux,
c) éventuellement on lave au shampooing et on rince lesdits cheveux,
d) on sèche ou on laisse sécher les cheveux.

Tout ce qui a été précédemment décrit concernant les divers éléments constitutifs de la composition reste valable et l'on pourra s'y reporter.

Notamment, les procédés selon l'invention sont appropriés pour traiter les cheveux pigmentées ou colorées artificiellement.
Plus particulièrement, les cheveux qui peuvent être avantageusement traitées par le procédé selon l'invention, présentent une hauteur de ton est inférieure ou égale à 6 (blond foncé) et de préférence inférieure ou égale à 4 (châtain).

Selon un premier mode de réalisation de l'invention, le procédé de coloration avec effet éclaircissant des cheveux est mis en oeuvre avec une composition ne comprenant pas de colorants d'oxydation ni de coupleur et en l'absence d'agent oxydant.

Selon un deuxième mode de réalisation de l'invention, le procédé de coloration avec effet éclaircissant des cheveux est mis en oeuvre avec une composition ne comprenant pas de colorants d'oxydation ni de coupleur, mais en présence d'agent(s) oxydant(s).

Selon une première variante de ces procédés de coloration conformes à l'invention, on applique sur les cheveux, au moins une composition telle que définie précédemment, pendant un temps suffisant pour développer la coloration et l'éclaircissement désirés, après quoi on rince, on lave éventuellement au shampooing, on rince à nouveau et on sèche.

Selon une deuxième variante de ces procédés de teinture conformes à l'invention, on applique sur les cheveux au moins une composition telle que définie précédemment sans rinçage final.

Selon une troisième variante de procédé de coloration conforme à l'invention, le procédé de teinture comporte une étape préliminaire consistant à stocker sous forme séparée, d'une part, une composition selon l'invention comprenant éventuellement au moins une base d'oxydation et/ou un coupleur et, d'autre part, une composition comprenant, dans un milieu cosmétiquement acceptable, au moins un agent oxydant, puis à procéder à leur mélange au moment de l'emploi avant d'appliquer ce mélange sur les cheveux, pendant un temps suffisant pour développer la coloration désirée, après quoi on rince les cheveux, on les lave éventuellement au shampooing, on les rince à nouveau et on les sèche.

Le temps nécessaire au développement de la coloration et à l'obtention de l'effet éclaircissant sur les cheveux, est d'environ 5 à 60 minutes et plus particulièrement d'environ 5 à 40 minutes.

La température nécessaire au développement de la coloration et à l'obtention de l'effet éclaircissant est généralement comprise entre la température ambiante (15 à 25°C) et 80°C et plus particulièrement entre 15 et 40°C.

Un autre objet de l'invention est un dispositif à plusieurs compartiments pour la coloration avec effet éclaircissant des fibres kératiniques et notamment des cheveux, comprenant au moins un compartiment renfermant une composition selon l'invention, et au moins un autre compartiment renfermant une composition comprenant au moins un agent oxydant. Ce dispositif peut être équipé d'un moyen permettant de délivrer sur les fibres le mélange souhaité, tel que les dispositifs décrits dans le brevet FR 2586913.

Il est à noter que la composition selon l'invention, si elle est utilisée pour traiter des fibres kératiniques, telles que des cheveux châtains par exemple, permet d'atteindre les résultats suivants :
Si l'on mesure la réflectance des cheveux lorsqu'on les irradie avec de la lumière visible dans la gamme de longueurs d'onde allant de 400 à 700 nanomètres, et que l'on compare les courbes de réflectance en fonction de la longueur d'onde, des cheveux traités avec la composition de l'invention et des cheveux non traités, on constate que la courbe de réflectance correspondant aux cheveux traités, dans une gamme de longueur d'onde allant de 500 à 700 nanomètres, est supérieure à celle correspondant aux cheveux non traités.
Cela signifie que, dans la gamme de longueur d'onde allant de 500 à 700 nanomètres, et de préférence de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%.
Il est précisé toutefois qu'il peut exister dans la gamme de longueur d'onde allant de 500 à 700 nanomètres, et de préférence de 540 à 700 nanomètres, une ou plusieurs plages où la courbe de réflectance correspondant aux fibres traitées est soit superposable, soit inférieure à la courbe de réflectance correspondant aux fibres non traitées.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

En outre, et de préférence, la composition selon l'invention est susceptible d'éclaircir les cheveux dans une nuance qui, chiffrée dans le système C.I.E.L L*a*b* présente une variable b* supérieure ou égale à 6, avec un rapport b*/valeur absolue de a*, supérieur à 1,2 selon le test de sélection décrit ci-dessous.

### Test de sélection

La composition est appliquée sur des fibres kératiniques châtain, plus particulièrement des cheveux, à raison de 10 grammes de composition pour 1 gramme de fibres châtain. La composition est étalée de façon à recouvrir l'ensemble des fibres. On laisse la composition agir pendant 20 minutes à température ambiante (20 à 25°C). Les fibres sont ensuite rincées à l'eau puis lavées avec un shampooing à base de lauryléther sulfate. Elles sont ensuite séchées. On mesure alors les caractéristiques spectrocolorimétriques des fibres pour en déterminer les coordonnées L*a*b*.
Dans le système C.I.E.L L*a*b*, a* et b* indiquent deux axes de couleurs, a* indique l'axe de couleur vert/rouge (+a* est rouge, -a* est vert) et b* l'axe de couleur bleu/jaune (+b* est jaune et -b* est bleu) ; des valeurs proches de zéro pour a* et b* correspondent à des nuances grises.

L'exemple qui suit est destiné à illustrer l'invention sans pour autant en limiter la portée.

### EXEMPLES

### Composé fluorescent

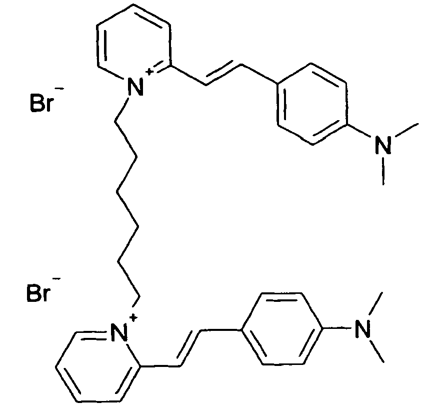

On fait réagir 93 g de 2-picoline avec 120g de 1,6 dibromohexane dans le diméthylformamide à 110°C pendant 5 heures.
On récupère le produit précipité, et on le filtre.
On solubilise 109 g du produit obtenu précédemment dans du méthanol et l'on ajoute 82,82 g de p-diméthylaminobenzaldéhyde en deux fois, en présence de pyrrolidine.
On laisse ensuite pendant 30 minutes.
On récupère le produit sous forme précipitée.

Analyse par spectroscopie de masse : 266.
Analyse élémentaire : C : 62,43 % ; H : 6,40 % ; Br : 23,07 % ; N : 8,09 %.
La formule est la suivante C₃₆H₄₄N₄.2Br.

### Compositions

On prépare les compositions suivantes :

| **Composition** | **1** | **2** |
|---|---|---|
| Composé fluorescent | 0,6 % | 0,6 % |
| Myristate d'isopropyle | - | 0,25 % |
| Arlamol HD (*) | 0,25 % | - |
| Lauryléther sulfate de sodium (2,2 OE) | 10 % | 10 % |
| Eau distillée | Qsp 100 % | Qsp 100 % |

| | | |
|---|---|---|
| pourcentages exprimés en poids de matière active (*) Arlamol HD : poly-α oléfine. | | |

Chaque composition est appliquée sur une mèche de cheveux naturels châtains de hauteur de ton 4, avec un temps de pose de 20minutes, un rinçage final et un séchage au casque pendant 30 minutes.

On obtient à chaque fois une mèche de cheveux avec un effet d'éclaircissement net.

## Revendications

1. Composition, **caractérisée en ce qu'**elle comprend, dans un milieu cosmétiquement acceptable, au moins un colorant fluorescent dans la famille des orangés soluble dans ledit milieu, au moins un agent conditionneur insoluble dans ledit milieu, choisi parmi les huiles de synthèse ; les huiles minérales ; les huiles végétales ; les huiles animales ; les huiles fluorées ou perfluorées ; les cires naturelles ou synthétiques ; les esters d'acides carboxyliques ; les composés de formule R₃CHOH-CH(NHCOR₁)-CH₂OR₂ dans laquelle R₁ est un radical alkyle en C₁₄-C₃₀, éventuellement substitué en position α par un radical hydroxyle, ou en position ω par un radical hydroxyle estérifié par un acide gras en C₁₆-C₃₀, R₂ désigne un atome d'hydrogène ou un radical (glycosyle)n ou (galactosyle)m avec n variant de 1 à 4 et m de 1 à 8, R₃ désigne un radical hydrocarboné en C₁₅-C₂₆ éventuellement substitué par un radical alkyle, ou un radical α-hydroxyalkyle en C₁₅-C₂₆ éventuellement estérifié par un α-hydroxyacide en C₁₆-C₃₀ ; seuls ou en mélanges.

2. Composition selon la revendication 1, **caractérisée en ce que** le colorant fluorescent conduit à un maximum de réflectance se situant dans la gamme de longueur d'onde allant de 500 à 650 nanomètres, et de préférence dans la gamme de longueur d'onde allant de 550 à 620 nanomètres.

3. Composition selon l'une quelconque des revendications précédentes, **caractérisée en ce que** le composé fluorescent est choisi parmi les colorants fluorescents appartenant aux familles suivantes : les naphtalimides ; les coumarines cationiques ou non ; les xanthénodiquinolizines ; les azaxanthènes ; les naphtolactames ; les azlactones ; les oxazines ; les thiazines ; les dioxazines ; les colorants fluorescents monocationiques ou polycationiques de type azoïque, azométhinique, ou méthinique, seuls ou en mélanges.

4. Composition selon l'une quelconque des revendications précédentes, **caractérisée en ce que** le composé fluorescent est de formule suivante : 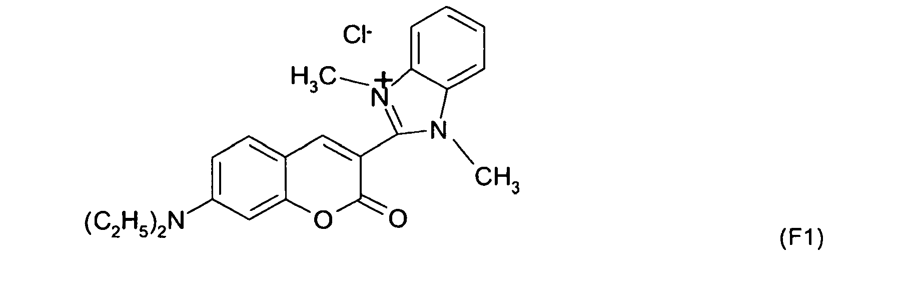 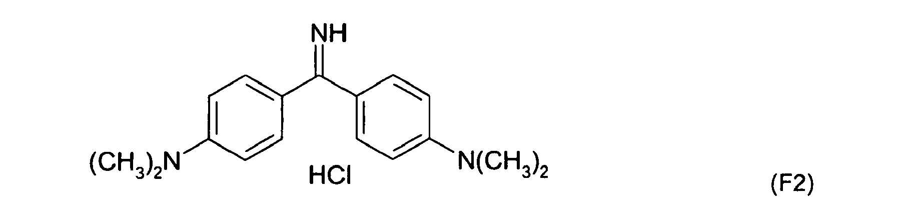 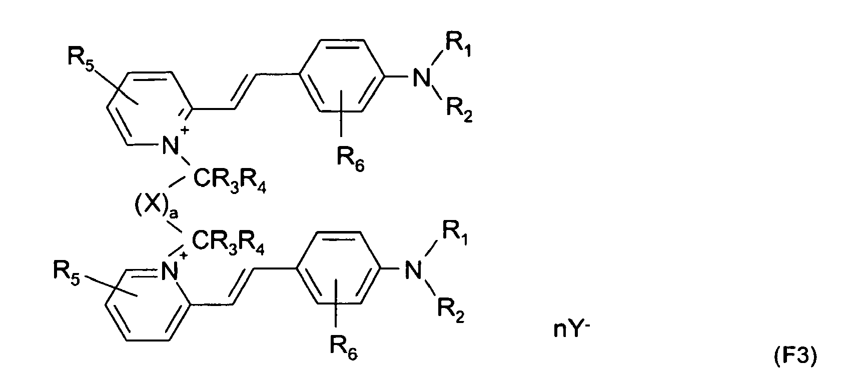 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent ; 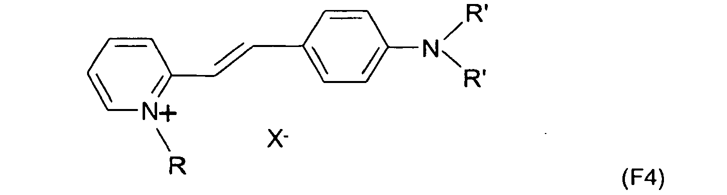 formule dans laquelle R représente un radical méthyle ou éthyle; R' représente un radical méthyle, X- un anion du type chlorure, iodure, sulfate, méthosulfate, acétate, perchlorate.

5. Composition selon l'une quelconque des revendications précédentes, **caractérisée en ce que** le ou les colorants fluorescents sont présents dans une concentration pondérale comprise entre 0,01 et 20% en poids, plus particulièrement comprise entre 0,05 à 10% en poids, de préférence comprise entre 0,1 à 5% en poids, par rapport au poids total de la composition.

6. Composition selon l'une quelconque des revendications précédentes, **caractérisée en ce que** l'agent conditionneur insoluble est choisi parmi
• Les huiles de synthèse comme les polyoléfines en particulier les poly-α-oléfines ;
• Les huiles animales ou végétales comme les huiles de tournesol, de maïs, de soja, d'avocat, de jojoba, de courge, de pépins de raisin, de sésame, de noisette, les huiles de poisson, le tricaprocaprylate de glycérol, les huiles végétales ou animales de formule R₉COOR₁₀ dans laquelle R₉ représente le reste d'un acide gras en C₇-C₂₉ et R₁₀ représente une chaîne hydrocarbonée linéaire ou ramifiée en C₃-C₃₀ ; les huiles essentielles naturelles ou synthétiques ;
• Les cires naturelles animales ou végétales, ou les cires synthétiques ;
• La N-linoléyldihydrosphingosine, la N-oléyldihydrosphingosine, la N-palmitoyldihydrosphingosine, la N-stéaroyldihydrosphingosine, la N-béhénoyldihydrosphingosine ;
• Les huiles fluorées comme les perfluoropolyéthers, les huiles fluorohydrocarbonées, les fluorocarbures, les hydrocarbures fluorés,
• Les alcools gras choisis parmi les alcools gras en C₈-C₂₂, linéaires ou ramifiés, éventuellement oxyalkylénés avec 1 à 15 moles d'oxyde d'alkylène ou polyglycérolés avec 1 à 6 moles de glycérol ;
• Les esters d'acides carboxyliques, comme les esters d'acides monocarboxyliques aliphatiques saturés ou insaturés, linéaires ou ramifiés en C₁-C₂₆ et d'alcools aliphatiques saturés, le nombre total de carbone des esters étant supérieur ou égal à 10 ; les mono-, di-, tri-, tétra ou penta- esters d'acides di- ou tricarboxyliques et d'alcools en C₁-C₂₂ ou d'alcools di-, tri-, tétra- ou penta-hydroxy en C₂-C₂₆ ;
seuls ou en mélange.

7. Composition selon l'une des revendications précédentes, **caractérisée en ce que** la teneur en agent conditionneur insoluble représente 0,01 à 20 % en poids par rapport au poids de la composition, plus particulièrement 0,1 à 10 % en poids par rapport à la même référence.

8. Composition selon l'une des revendications précédentes, **caractérisée en ce qu**'elle comprend au moins un tensioactif non ionique, anionique ou amphotère.

9. Composition selon la revendication précédente, **caractérisée en ce que** la teneur en tensioactif représente 0,01 à 30 % en poids par rapport au poids total de la composition.

10. Composition selon l'une quelconque des revendications précédentes, **caractérisée en ce qu**'elle comprend en outre au moins un colorant direct non fluorescent additionnel de nature non ionique, cationique ou anionique.

11. Composition selon la revendication 10, **caractérisée par le fait que** les colorants directs additionnels sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ainsi que les colorants dérivés du triarylméthane, ou leurs mélanges.

12. Composition selon l'une des revendications 10 ou 11, **caractérisée en ce que** le ou les colorants directs additionnels représentent de 0,0005 à 12 % en poids, de préférence de 0,005 à 6 % en poids, du poids total de la composition.

13. Composition selon l'une quelconque des revendications précédentes, **caractérisée en ce qu**'elle se présente sous la forme d'un shampooing éclaircissant et colorant.

14. Composition selon l'une quelconque des revendications précédentes, **caractérisée en ce qu**'elle comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcalin.

15. Composition selon la revendication précédente, **caractérisée en ce que** la ou les bases d'oxydation représentent 0,0005 à 12 % en poids, plus particulièrement 0,005 à 6 % en poids, du poids total de la composition.

16. Composition selon l'une quelconque des revendications 14 ou 15, **caractérisée en ce qu**'elle comprend au moins un coupleur choisi parmi les métaphénylène diamines, les méta-aminophénols, les métadiphénols et les coupleurs hétérocycliques ou leurs sels d'addition avec un acide ou avec un agent alcalin.

17. Composition selon la revendication précédente, **caractérisée en ce que** le ou les coupleurs représentent de 0,0001 à 10 % en poids, plus particulièrement 0,005 à 5 % en poids, du poids total de la composition tinctoriale.

18. Composition, **caractérisée en ce qu'**elle comprend la composition selon l'une des revendications 1 à 12 et 14 à 17, et au moins un agent oxydant.

19. Composition selon la revendication précédente, **caractérisée en ce que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à deux ou quatre électrons, et de préférence le peroxyde d'hydrogène.

20. Procédé pour colorer avec un effet éclaircissant les cheveux présentant une hauteur de ton inférieure ou égale à 6, **caractérisé en ce que** l'on met en oeuvre les étapes suivantes :
a) on applique sur lesdits cheveux une composition telle que définie selon l'une quelconque des revendications 1 à 19, pendant un temps suffisant pour développer la coloration et l'éclaircissement désirés,
b) on rince éventuellement les cheveux,
c) éventuellement on lave au shampooing et on rince les cheveux,
d) on sèche ou on laisse sécher les cheveux.

21. Procédé selon la revendication 20, **caractérisé en ce qu**'il comporte une étape préliminaire consistant à stocker sous forme séparée, d'une part, une composition selon l'une des revendications 1 à 12 et 14 à 17, et d'autre part, une composition renfermant, dans un milieu cosmétiquement acceptable, au moins un agent oxydant, puis à procéder à leur mélange au moment de l'emploi avant d'appliquer ce mélange sur les cheveux pendant un temps suffisant pour développer la coloration désirée, après quoi on les rince, on les lave éventuellement au shampooing, on les rince à nouveau et on les sèche.

22. Procédé selon l'une quelconque des revendications 20 ou 21, **caractérisé par le fait que** la composition est appliquée sur des cheveux présentant une hauteur de ton inférieure ou égale à 4.

23. Procédé selon l'une des revendications 20 à 22, **caractérisé en ce que** les cheveux sont pigmentés ou colorés artificiellement.

24. Dispositif à plusieurs compartiments pour la teinture et l'éclaircissement des fibres kératiniques, comprenant au moins un compartiment renfermant une composition selon l'une des revendications 1 à 12 et 14 à 17, et au moins un autre compartiment renfermant une composition renfermant au moins un agent oxydant.

25. Utilisation d'une composition telle que définie dans l'une quelconque des revendications 1 à 19 pour la coloration avec effet éclaircissant de cheveux présentant une hauteur de ton inférieure ou égale à 6.

26. Utilisation selon la revendication 25, **caractérisée par le fait que** les cheveux sont des cheveux pigmentés ou colorés artificiellement.

27. Utilisation selon la revendication 26, **caractérisée par le fait que** les cheveux présentent une hauteur de ton inférieure ou égale à 4.

## Claims

1. Composition, **characterized in that** it comprises, in a cosmetically acceptable medium, at least one dye which is fluorescent in the orange range and which is soluble in the said medium, at least one conditioning agent which is insoluble in the said medium and which is chosen from synthetic oils; mineral oils; vegetable oils; animal oils; fluorinated or perfluorinated oils; natural or synthetic waxes; carboxylic acid esters; compounds of formula R₃CHOH-CH (NHCOR₁) -CH₂OR₂ in which R₁ is a C₁₉-C₃₀ alkyl radical optionally substituted in the α position by a hydroxyl radical or in the ω position by a hydroxyl radical esterified by a C₁₆-C₃₀ fatty acid, R₂ denotes a hydrogen atom or a (glycosyl)ₙ or (galactosyl)ₘ radical with n varying from 1 to 4 and m from 1 to 8, and R₃ denotes a C₁₅-C₂₆ hydrocarbon radical optionally substituted by an alkyl radical or a C₁₅-C₂₆ α-hydroxyalkyl radical optionally esterified by a C₁₆-C₃₀ α-hydroxy acid; alone or as mixtures.

2. Composition according to Claim 1, **characterized in that** the fluorescent dye results in a maximum of reflectance lying in the wavelength range extending from 500 to 650 nanometers and preferably in the wavelength range extending from 550 to 620 nanometers.

3. Composition according to either one of the preceding claims, **characterized in that** the fluorescent compound is chosen from fluorescent dyes belonging to the following families: naphthalimides; cationic or noncationic coumarins; xanthenodiquinolizines; azaxanthenes; naphtholactams; azlactones; oxazines; thiazines; dioxazines; monocationic or polycationic fluorescent dyes of azo, azomethine or methine type, alone or as mixtures.

4. Composition according to any one of the preceding claims, **characterized in that** the fluorescent compound is of following formula: 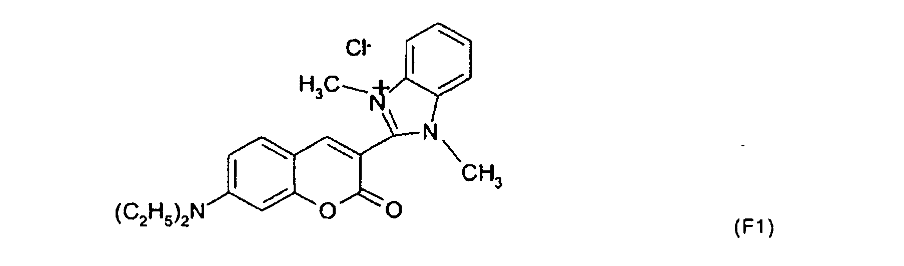 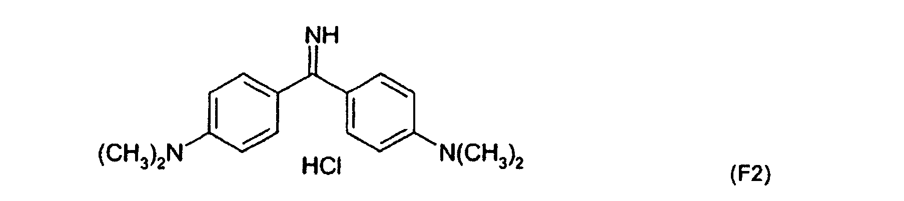 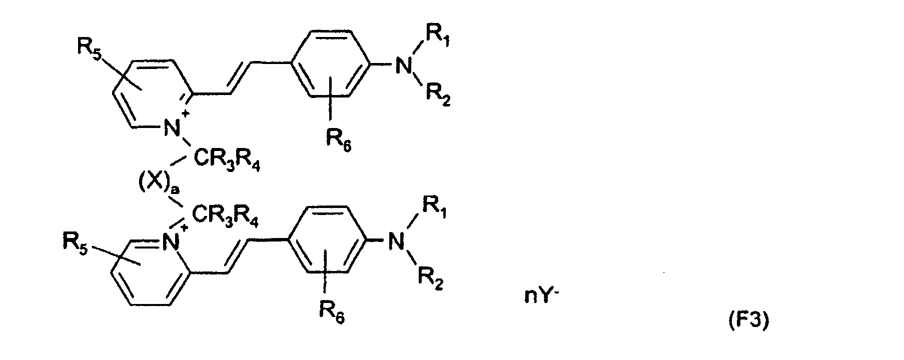 in which:
R₁ and R₂, which are identical or different, represent:
• a hydrogen atom;
• a linear or branched alkyl radical comprising 1 to 10 carbon atoms, preferably from 1 to 4 carbon atoms, which is optionally interrupted and/or substituted by at least one heteroatom and/or group comprising at least one heteroatom and/or substituted by at least one halogen atom;
• an aryl or arylalkyl radical, the aryl group having 6 carbon atoms and the alkyl radical having 1 to 4 carbon atoms; the aryl radical optionally being substituted by one or more linear or branched alkyl radicals comprising 1 to 4 carbon atoms which are optionally interrupted and/or substituted by at least one heteroatom and/or group comprising at least one heteroatom and/or substituted by at least one halogen atom;
• R₁ and R₂ can optionally be connected so as to form a heterocycle with the nitrogen atom and can comprise one or more other heteroatoms, the heterocycle optionally being substituted by at least one linear or branched alkyl radical preferably comprising from 1 to 4 carbon atoms and optionally being interrupted and/or substituted by at least one heteroatom and/or group comprising at least one heteroatom and/or substituted by at least one halogen atom;
• R₁ or R₂ can optionally be involved in a heterocycle comprising the nitrogen atom and one of the carbon atoms of the phenyl group carrying the said nitrogen atom;
R₃ and R₄, which are identical or different, represent a hydrogen atom or an alkyl radical comprising 1 to 4 carbon atoms;
R₅, which are identical or different, represent a hydrogen atom, a halogen atom or a linear or branched alkyl radical comprising 1 to 4 carbon atoms which is optionally interrupted by at least one heteroatom;
R₆, which are identical or different, represent a hydrogen atom, a halogen atom or a linear or branched alkyl radical comprising 1 to 4 carbon atoms which is optionally substituted and/or interrupted by at least one heteroatom and/or group carrying at least one heteroatom and/or substituted by at least one halogen atom;
X represents:
• a linear or branched alkyl radical comprising 1 to 14 carbon atoms or an alkenyl radical comprising 2 to 14 carbon atoms which is optionally interrupted and/or substituted by at least one heteroatom and/or group comprising at least one heteroatom and/or substituted by at least one halogen atom;
• a heterocyclic radical comprising 5 or 6 ring members, optionally substituted by at least one linear or branched alkyl radical comprising 1 to 14 carbon atoms, optionally substituted by at least one heteroatom; by at least one linear or branched aminoalkyl radical comprising 1 to 4 carbon atoms, optionally substituted by at least one heteroatom; by at least one halogen atom;
• an aromatic radical or a diaromatic radical which is or is not fused and which is or is not separated by an alkyl radical comprising 1 to 4 carbon atoms, the aryl radical or radicals optionally being substituted by at least one halogen atom or by at least one alkyl radical comprising 1 to 10 carbon atoms which is optionally substituted and/or interrupted by at least one heteroatom and/or group carrying at least one heteroatom;
• a dicarbonyl radical;
• it being possible for the group X to carry one or more cationic charges;
a is equal to 0 or 1;
Y⁻, which are identical or different, represent an organic or inorganic anion;
n is an integer at least equal to 2 and at most equal to the number of cationic charges present in the fluorescent compound: 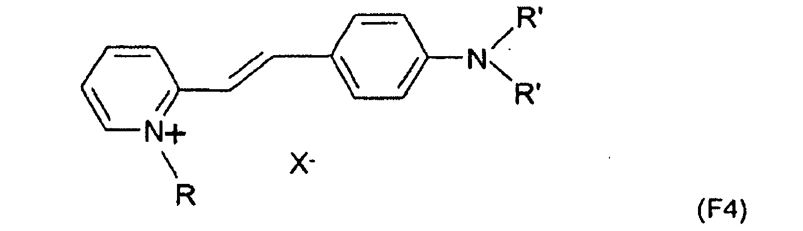
in which formula R represents a methyl or ethyl radical; R' represents a methyl radical and X⁻ represents an anion of the chloride, iodide, sulphate, methosulphate, acetate or perchlorate type.

5. Composition according to any one of the preceding claims, **characterized in that** the fluorescent dye or dyes are present in a concentration by weight of between 0.01 and 20% by weight, more particularly of between 0.05 and 10% by weight, preferably of between 0.1 and 5% by weight, with respect to the total weight of the composition.

6. Composition according to any one of the preceding claims, **characterized in that** the insoluble conditioning agent is chosen from:
• synthetic oils, such as polyolefins, in particular poly-α-olefins;
• animal or vegetable oils, such as sunflower, maize, soybean, avocado, jojoba, cucumber, grape seed, sesame or hazelnut oils, fish oils, glycerol tricaprate/caprylate, vegetable or animal oils of formula R₉COOR₁₀ in which R₉ represents the residue of a C₇-C₂₉ fatty acid and R₁₀ represents a linear or branched C₃-C₃₀ hydrocarbon chain; natural or synthetic essential oils;
• natural animal or vegetable waxes, or synthetic waxes;
• N-linoleoyldihydrosphingosine, N-oleoyldihydrosphingosine, N-palmitoyldihydrosphingosine, N-stearoyldihydrosphingosine or N-behenoyldihydrosphingosine;
• fluorinated oils, such as perfluoropolyethers, fluorohydrocarbon oils, fluorocarbons or fluorinated hydrocarbons;
• fatty alcohols chosen from linear or branched C₈-C₂₂ fatty alcohols which are optionally oxyalkylenated with 1 to 15 mol of alkylene oxide or polyglycerolated with 1 to 6 mol of glycerol;
• carboxylic acid esters, such as esters of saturated or unsaturated and linear or branched aliphatic C₁-C₂₆ monocarboxylic acids and of saturated aliphatic alcohols, the total carbon number of the esters being greater than or equal to 10; mono-, di-, tri-, tetra- or pentaesters of di- or tricarboxylic acids and of C₁-C₂₂ alcohols or of di-, tri-, tetra- or pentahydroxy C₂-C₂₆ alcohols;
alone or as mixtures.

7. Composition according to one of the preceding claims, **characterized in that** the content of insoluble conditioning agent represents 0.01 to 20% by weight, with respect to the weight of the composition, more particularly 0.1 to 10% by weight, with respect to the same reference.

8. Composition according to one of the preceding claims, **characterized in that** it comprises at least one nonionic, anionic or amphoteric surfactant.

9. Composition according to the preceding claim, **characterized in that** the content of surfactant represents 0.01 to 30% by weight, with respect to the total weight of the composition.

10. Composition according to any one of the preceding claims, **characterized in that** it additionally comprises at least one additional nonfluorescent direct dye of nonionic, cationic or anionic nature.

11. Composition according to Claim 10, **characterized in that** the additional direct dyes are chosen from nitrobenzene dyes, azo, anthraquinone, naphthoquinone, benzoquinone, phenothiazine, indigoid, xanthene, phenanthridine or phthalocyanine dyes, and dyes derived from triarylmethane, or their mixtures.

12. Composition according to either of Claims 10 and 11, **characterized in that** the additional direct dye or dyes represent from 0.0005 to 12% by weight, preferably from 0.005 to 6% by weight, of the total weight of the composition.

13. Composition according to any one of the preceding claims, **characterized in that** it is provided in the form of a lightening and colouring shampoo.

14. Composition according to any one of the preceding claims, **characterized in that** it comprises at least one oxidation base chosen from paraphenylenediamines, bisphenylalkylenediamines, para-aminophenols, ortho-aminophenols and heterocyclic bases or their addition salts with an acid or with an alkaline agent.

15. Composition according to the preceding claim, **characterized in that** the oxidation base or bases represent from 0.0005 to 12% by weight, more particularly from 0.005 to 6% by weight, of the total weight of the composition.

16. Composition according to either one of Claims 14 and 15, **characterized in that** it comprises at least one coupler chosen from metaphenylenediamines, meta-aminophenols, metadiphenols and heterocyclic couplers or their addition salts with an acid or with an alkaline agent.

17. Composition according to the preceding claim, **characterized in that** the coupler or couplers represent from 0.0001 to 10% by weight, more particularly from 0.005 to 5% by weight, of the total weight of the dyeing composition.

18. Composition, **characterized in that** it comprises the composition according to one of Claims 1 to 12 and 14 to 17 and at least one oxidizing agent.

19. Composition according to the preceding claim, **characterized in that** the oxidizing agent is chosen from hydrogen peroxide, urea hydrogen peroxide, alkali metal bromates, persalts, such as perborates and persulphates, and enzymes, such as peroxidases and two- or four-electron oxidoreductases, and preferably hydrogen peroxide.

20. Method for colouring with a lightening effect hair exhibiting a tone depth of less than or equal to 6, **characterized in that** the following stages are carried out:
a) a composition as defined according to any one of Claims 1 to 19 is applied to the said hair for a time sufficient to develop the desired colouring and the desired lightening,
b) the hair is optionally rinsed,
c) the hair is optionally washed with a shampoo and rinsed,
d) the hair is dried or allowed to dry.

21. Method according to Claim 20, **characterized in that** it comprises a preliminary stage which consists in storing, in separate form, on the one hand, a composition according to one of Claims 1 to 12 and 14 to 17 and, on the other hand, a composition including, in a cosmetically acceptable medium, at least one oxidizing agent, and in then mixing them at the time of use before applying this mixture to the hair for a time sufficient to develop the desired colouring, after which the hair is rinsed, optionally washed with a shampoo, again rinsed and dried.

22. Method according to either one of Claims 20 and 21, **characterized in that** the composition is applied to hair exhibiting a tone depth of less than or equal to 4.

23. Method according to one of Claims 20 to 22, **characterized in that** the hair is artificially pigmented or coloured.

24. Multicompartment device for the dyeing and lightening of keratinous fibres, comprising at least one compartment including a composition according to one of Claims 1 to 12 and 14 to 17 and at least one other compartment including a composition including at least one oxidizing agent.

25. Use of a composition as defined in any one of Claims 1 to 19 for the colouring with a lightening effect of hair exhibiting a tone depth of less than or equal to 6.

26. Use according to Claim 25, **characterized in that** the hair is artificially pigmented or coloured hair.

27. Use according to Claim 26, **characterized in that** the hair exhibits a tone depth of less than or equal to 4.

## Patentansprüche

1. Zusammensetzung, **dadurch gekennzeichnet, dass** sie in einem kosmetisch akzeptablen Medium mindestens einen im Orangebereich fluoreszierenden Farbstoff, der in dem Medium löslich ist, und mindestens ein Konditioniermittel enthält, das in dem Medium unlöslich ist und einzeln oder in Form von Gemischen ausgewählt ist unter synthetischen Ölen; Mineralölen; pflanzlichen Ölen; tierischen Ölen; fluorierten oder perfluorierten Ölen; natürlichen oder synthetischen Wachsen; Carbonsäureestern; Verbindungen der Formel R₃CHOH-CH(NHCOR₁)-CH₂OR₂, worin R₁ eine C₁₄₋₃₀-Alkylgruppe bedeutet, die gegebenenfalls in α-Stellung mit einer Hydroxygruppe oder in ω-Stellung mit einer Hydroxygruppe substituiert ist, die mit einer C₁₆₋₃₀-Fettsäure verestert ist, R₂ ein Wasserstoffatom oder eine Gruppe (Glycosyl)n oder (Galactosyl)m bedeutet, wobei n im Bereich von 1 bis 4 und m im Bereich von 1 bis 8 liegt, und R₃ eine C₁₅₋₂₆-Kohlenwasserstoffgruppe, die gegebenenfalls mit einer Alkylgruppe substituiert ist, oder eine C₁₅₋₂₆-α-Hydroxyalkylgruppe bedeutet, die gegebenenfalls mit einer C₁₆₋₃₀-α-Hydroxysäure verestert ist.

2. Zusammensetzung nach Anspruch 1, **dadurch gekennzeichnet, dass** der fluoreszierende Farbstoff im Wellenlängenbereich von 500 bis 650 nm und vorzugsweise im Wellenlängenbereich von 550 bis 620 nm zu einem maximalen Reflexionsvermögen führt.

3. Zusammensetzung nach einem der vorhergehenden Ansprüche, **dadurch gekennzeichnet, dass** die fluoreszierende Verbindung einzeln oder in Form von Gemischen unter den fluoreszierenden Farbstoffen der folgenden Gruppen ausgewählt ist: Naphthalimiden; kationischen oder nicht kationischen Cumarinen; Xanthenodichinolizinen; Azaxanthenen; Naphtholactamen; Azlactonen; Oxazinen; Thiazinen; Dioxazinen; monokationischen oder polykationischen fluoreszierenden Farbstoffen vom Azotyp, Azomethintyp oder Methintyp.

4. Zusammensetzung nach einem der vorhergehenden Ansprüche, **dadurch gekennzeichnet, dass** die fluoreszierende Verbindung die folgende Formel aufweist: 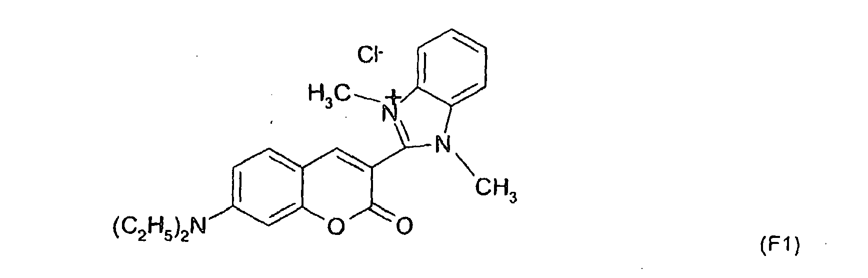 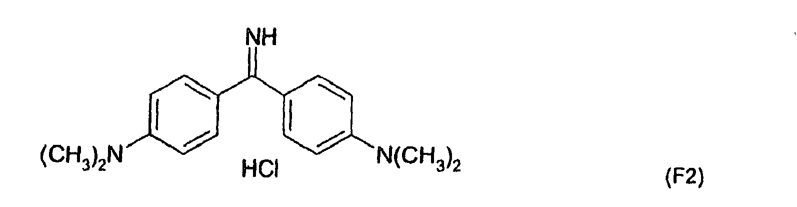 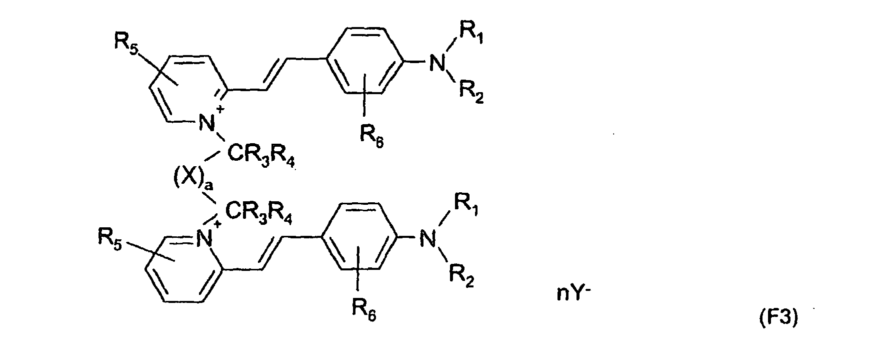 wobei in der Formel bedeuten:
R₁, R₂, die gleich oder verschieden sind, bedeuten:
• ein Wasserstoffatom;
• eine geradkettige oder verzweigte Alkylgruppe mit 1 bis 10 Kohlenstoffatomen und vorzugsweise 1 bis 4 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
• eine Aryl- oder Arylalkylgruppe, wobei die Arylgruppe 6 Kohlenstoffatome besitzt und die Alkylgruppe 1 bis 4 Kohlenstoffatome aufweist; wobei die Arylgruppe gegebenenfalls substituiert ist mit einer oder mehreren geradkettigen oder verzweigten Alkylgruppen mit 1 bis 4 Kohlenstoffatomen, die gegebenenfalls mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert sind, und/oder mit mindestens einem Halogenatom;
• R₁ und R₂ können gegebenenfalls so verbunden sein, dass sie mit dem Stickstoffatom einen Heterocyclus bilden und sie können ein oder mehrere weitere Heteroatome enthalten, wobei der Heterocyclus gegebenenfalls mit mindestens einer geradkettigen oder verzweigten Alkylgruppe substituiert ist, die vorzugsweise 1 bis 4 Kohlenstoffatome enthält und gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
• R₁ oder R₂ können gegebenenfalls in einen Heterocyclus eingebunden sein, der das Stickstoffatom und eines der Kohlenstoffatome des Phenylrings enthält, der das Stickstoffatom trägt;
R₃, R₄, die gleich oder verschieden sind, bedeuten ein Wasserstoffatom, eine Alkylgruppe mit 1 bis 4 Kohlenstoffatomen;
die Gruppen R₅, die gleich oder verschieden sind, bedeuten ein Wasserstoffatom, ein Halogenatom, eine geradkettige oder verzweigte Alkylgruppe mit 1 bis 4 Kohlenstoffatomen, die gegebenenfalls durch mindestens ein Heteroatom unterbrochen ist;
die Gruppen R₆, die gleich oder verschieden sind, bedeuten ein Wasserstoffatom; und ein Halogenatom; eine geradkettige oder verzweigte Alkylgruppe mit 1 bis 4 Kohlenstoffatomen, die gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist und/oder mit mindestens einem Halogenatom substituiert ist;
X bedeutet:
• eine geradkettige oder verzweigte Alkylgruppe mit 1 bis 14 Kohlenstoffatomen oder eine Alkenylgruppe mit 2 bis 14 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert sind und/oder mit mindestens einem Halogenatom substituiert sind;
• eine 5- oder 6-gliedrige heterocyclische Gruppe, die gegebenenfalls substituiert ist mit mindestens einer geradkettigen oder verzweigten Alkylgruppe mit 1 bis 14 Kohlenstoffatomen, die gegebenenfalls mit mindestens einem Heteroatom substituiert ist; mit mindestens einer geradkettigen oder verzweigten Aminoalkylgruppe mit 1 bis 4 Kohlenstoffatomen, die gegebenenfalls mit mindestens einem Heteroatom substituiert ist; mit mindestens einem Halogenatom;
• eine aromatische oder kondensierte oder nichtkondensierte diaromatische Gruppe, die gegebenenfalls durch eine Alkylgruppe mit 1 bis 4 Kohlenstoffatomen getrennt ist, wobei die Arylgruppe(n) gegebenenfalls mit mindestens einem Halogenatom oder mit mindestens einer Alkylgruppe substituiert sind, die 1 bis 10 Kohlenstoffatome aufweist und gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist;
• eine Dicarbonylgruppe;
• wobei die Gruppe X eine oder mehrere kationische Ladungen aufweisen kann;
a bedeutet 0 oder 1;
die Gruppen Y⁻, die gleich oder verschieden sind, bedeuten ein organisches oder anorganisches Anion;
wobei n eine ganze Zahl von mindestens 2 und höchstens der Anzahl der kationischen Ladungen, die an der fluoreszierenden Verbindung vorliegen, ist; 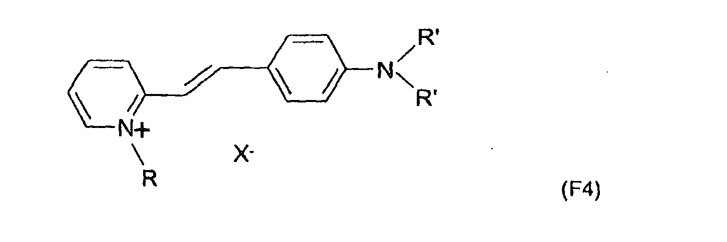 wobei in der Formel R Methyl oder Ethyl bedeutet; R' eine Methylgruppe ist; und X- ein Anion vom Typ Chlorid, Iodid, Sulfat, Methosulfat, Acetat, Perchlorat ist.

5. Zusammensetzung nach einem der vorhergehenden Ansprüche, **dadurch gekennzeichnet, dass** der oder die fluoreszierenden Farbstoffe in einer Konzentration von 0,01 bis 20 Gew.-%, insbesondere 0,05 bis 10 Gew.-% und vorzugsweise 0,1 bis 5 Gew.-%, bezogen auf das Gesamtgewicht der Zusammensetzung, enthalten sind.

6. Zusammensetzung nach einem der vorhergehenden Ansprüche, **dadurch gekennzeichnet, dass** das unlösliche Konditioniermittel ausgewählt ist unter:
• synthetischen Ölen, wie Polyolefinen und insbesondere Poly-α-olefinen;
• tierischen oder pflanzlichen Ölen, wie Sonnenblumenöl, Maisöl, Sojaöl, Avocadoöl, Jojobaöl, Kürbiskernöl, Traubenkernöl, Sesamöl, Haselnussöl, Fischölen, Glyceryltricaprocaprylat, pflanzlichen oder tierischen Ölen der Formel R₉COOR₁₀, wobei R₉ den Rest einer C₇₋₂₉-Fettsäure und R₁₀ eine geradkettige oder verzweigte C₃₋₃₀-Kohlenwasserstoffkette bedeutet; natürlichen oder synthetischen etherischen Ölen;
• natürlichen, tierischen oder pflanzlichen Wachsen oder synthetischen Wachsen;
• N-Linoleyldihydrosphingosin, N-Oleyldihydrosphingosin, N-Palmitoyldihydrosphingosin, N-Stearoyldihydrosphingosin, N-Behenoyldihydrosphingosin;
• fluorierten Ölen, wie Perfluorpolyethern, Fluorkohlenwasserstoffölen, Fluorkohlenstoffen, fluorierten Kohlenwasserstoffen;
• Fettalkoholen, die unter den geradkettigen oder verzweigten, gegebenenfalls mit 1 bis 15 mol Alkylenoxid alkoxylierten oder mit 1 bis 6 mol Glycerin mehrfach veretherten C₈₋₂₂-Fettalkoholen ausgewählt sind;
• Carbonsäureestern, beispielsweise Estern von gesättigten oder ungesättigten, geradkettigen oder verzweigten C₁₋₂₆-Monocarbonsäuren und gesättigten aliphatischen Alkoholen, wobei die Gesamtzahl der Kohlenstoffatome der Ester mindestens 10 beträgt; Mono-, Di-, Tri-, Tetra- oder Pentaestern von Di- oder Tricarbonsäuren und Alkoholen mit 1 bis 22 Kohlenstoffatomen oder Di-, Tri-, Tetra- oder Penta-Hydroxyalkoholen mit 2 bis 26 Kohlenstoffatomen;
einzeln oder in Form von Gemischen.

7. Zusammensetzung nach einem der vorhergehenden Ansprüche, **dadurch gekennzeichnet, dass** der Gehalt an unlöslichen Konditioniermitteln 0,01 bis 20 Gew.-%, bezogen auf das Gewicht der Zusammensetzung, und insbesondere 0,1 bis 10 Gew.-%, bezogen auf die gleiche Referenz, ausmacht.

8. Zusammensetzung nach einem der vorhergehenden Ansprüche, **dadurch gekennzeichnet, dass** sie mindestens einen nichtionischen, anionischen oder amphoteren grenzflächenaktiven Stoff enthält.

9. Zusammensetzung nach dem vorhergehenden Anspruch, **dadurch gekennzeichnet, dass** der Mengenanteil des grenzflächenaktiven Stoffes 0,01 bis 30 Gew.-%, bezogen auf das Gesamtgewicht der Zusammensetzung, ausmacht.

10. Zusammensetzung nach einem der vorhergehenden Ansprüche, **dadurch gekennzeichnet, dass** sie ferner mindestens einen ergänzenden nichtfluoreszierenden Direktfarbstoff vom nichtionischen, kationischen oder anionischen Typ enthält.

11. Zusammensetzung nach Anspruch 10, **dadurch gekennzeichnet, dass** die ergänzenden Direktfarbstoffe unter den nitrierten Benzolfarbstoffen, Azofarbstoffen, Anthrachinon-Farbstoffen, Naphthochinon-Farbstoffen, Benzochinon-Farbstoffen, Phenotiazin-Farbstoffen, Indigoiden, Xanthen-Farbstoffen, Phenanthridin-Farbstoffen, Phthalocyaninen sowie den von Triarylmethan abgeleiteten Farbstoffen oder deren Gemischen ausgewählt sind.

12. Zusammensetzung nach den Ansprüchen 10 oder 11, **dadurch gekennzeichnet, dass** der oder die ergänzenden Direktfarbstoffe 0,0005 bis 12 Gew.-% und vorzugsweise 0,005 bis 6 Gew.-% des Gesamtgewichts der Zusammensetzung ausmachen.

13. Zusammensetzung nach einem der vorhergehenden Ansprüche, **dadurch gekennzeichnet, dass** sie als aufhellendes und färbendes Haarwaschmittel vorliegt.

14. Zusammensetzung nach einem der vorhergehenden Ansprüche, **dadurch gekennzeichnet, dass** sie ferner mindestens eine Oxidationsbase enthält, die unter den *p*-Phenylendiaminen, Bisphenylalkylendiaminen, *p*-Aminophenolen, *o*-Aminophenolen und heterocyclischen Basen oder ihren Additionssalzen mit einer Säure oder mit einem alkalischen Stoff ausgewählt ist.

15. Zusammensetzung nach dem vorhergehenden Anspruch, **dadurch gekennzeichnet, dass** die Oxidationsbase(n) 0,0005 bis 12 Gew.-% und insbesondere 0,005 bis 6 Gew.-% des Gesamtgewichts der Zusammensetzung ausmachen.

16. Zusammensetzung nach einem der Ansprüche 14 oder 15, **dadurch gekennzeichnet, dass** sie ferner mindestens einen Kuppler enthält, der unter den *m*-Phenylendiaminen, *m*-Aminophenolen, *m*-Dihydroxybenzolen und heterocyclischen Kupplern oder deren Additionssalzen mit einer Säure oder einem alkalischen Stoff ausgewählt ist.

17. Zusammensetzung nach dem vorhergehenden Anspruch, **dadurch gekennzeichnet, dass** der oder die Kuppler 0,0001 bis 10 Gew.-% und insbesondere 0,005 bis 5 Gew.-% des Gesamtgewichts der Farbmittelzusammensetzung ausmachen.

18. Zusammensetzung, **dadurch gekennzeichnet, dass** sie die Zusammensetzung nach einem der Ansprüche 1 bis 12 und 14 bis 17 und mindestens ein Oxidationsmittel enthält.

19. Zusammensetzung nach dem vorhergehenden Anspruch, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten und Enzymen, wie Peroxidasen und Oxidoreductasen mit 2 oder 4 Elektronen und vorzugsweise Wasserstoffperoxid ausgewählt ist.

20. Verfahren zum Färben von Haaren, die einen Farbton von 6 oder darunter aufweisen, mit einer aufhellenden Wirkung, **dadurch gekennzeichnet, dass** die folgenden Schritte durchgeführt werden:
a) auf die Haare wird eine Zusammensetzung, wie sie in einem der Ansprüche 1 bis 19 definiert ist, während einer Zeitspanne aufgebracht, die ausreichend ist, um die gewünschte Färbung und Aufhellung zu bilden,
b) die Haare werden gegebenenfalls gespült,
c) die Haare werden gegebenenfalls mit Haarwaschmittel gewaschen und gespült,
d) die Haare werden getrocknet oder trocknen gelassen.

21. Verfahren nach Anspruch 20, **dadurch gekennzeichnet, dass** es einen vorbereitenden Schritt umfasst, der darin besteht, eine Zusammensetzung nach einem der Ansprüche 1 bis 12 und 14 bis 17 und eine Zusammensetzung getrennt voneinander aufzubewahren, die in einem kosmetisch akzeptablen Medium mindestens ein Oxidationsmittel enthält, sie dann bei der Anwendung zu vermischen, bevor das Gemisch auf die Haare während einer Zeitspanne aufgebracht wird, die ausreichend ist, um die gewünschte Färbung zu bilden, worauf gespült, gegebenenfalls mit Haarwaschmittel gewaschen und nochmals gespült und getrocknet wird.

22. Verfahren nach einem der Ansprüche 20 oder 21, **dadurch gekennzeichnet, dass** die Zusammensetzung auf Haare aufgebracht wird, die einen Farbton von höchstens 4 aufweisen.

23. Verfahren nach einem der Ansprüche 20 bis 22, **dadurch gekennzeichnet, dass** es sich bei den Haaren um künstlich gefärbte oder pigmentierte Haare handelt.

24. Vorrichtung mit mehreren Abteilungen zum Färben und Aufhellen von Keratinfasern, die mindestens eine Abteilung aufweist, die eine Zusammensetzung nach einem der Ansprüche 1 bis 12 und 14 bis 17 enthält und mindestens eine andere Abteilung, die eine Zusammensetzung enthält, die mindestens ein Oxidationsmittel umfasst.

25. Verwendung einer Zusammensetzung nach einem der Ansprüche 1 bis 19 zum Färben von Haaren, die einen Farbton von 6 oder darunter aufweisen, mit aufhellender Wirkung.

26. Verwendung nach Anspruch 25, **dadurch gekennzeichnet, dass** es sich bei den Haaren um künstlich gefärbte oder pigmentierte Haare handelt.

27. Verwendung nach Anspruch 26, **dadurch gekennzeichnet, dass** die Haare einen Farbton von 4 oder darunter aufweisen.
